# EUROPÄISCHE PATENTSCHRIFT

(11) **EP 4 143 289 B1**
(45) Veröffentlichungstag und Bekanntmachung des Hinweises auf die Patenterteilung: **06.05.2026**
(21) Anmeldenummer: 21714140.7
(22) Anmeldetag: 23.03.2021
(51) Int. Cl.: C11D 3/386

(54) **HOCHALKALISCHES TEXTILWASCHMITTEL MIT PROTEASE**
HIGHLY ALKALINE TEXTILE WASHING AGENT COMPRISING PROTEASE
DÉTERGENT POUR TEXTILES FORTEMENT ALCALIN CONTENANT UNE PROTÉASE

(30) Priorität: 29.04.2020 DE 102020205400
(43) Veröffentlichungstag der Anmeldung: 08.03.2023
(73) Patentinhaber: Henkel AG & Co. KGaA, 40589 Düsseldorf (DE)
(72) Erfinder: DEGERING, Christian, 40699 Erkrath (DE); WIELAND, Susanne, 41541 Zons/Dormagen (DE); LINDNER, Claudia, 42719 Solingen (DE); ISLAM, Shohana, 53359 Rheinbach (DE)
(86) Internationale Anmeldenummer: PCT/EP2021/057353
(87) Internationale Veröffentlichungsnummer: WO 2021/219296

(56) Entgegenhaltungen:
- EP-A1- 2 357 220
- WO-A1-2019/048495
- WO-A1-2020/221579
- WO-A1-2020/221580
- WO-A2-2013/063460

## Beschreibung

Die Erfindung liegt auf dem Gebiet der Waschmittel, insbesondere Waschmittel mit mindestens einem Enzym. Die Erfindung betrifft ein Textilwaschmittel, insbesondere ein flüssiges Textilwaschmittel, umfassend mindestens eine Protease, deren Aminosäuresequenz insbesondere im Hinblick auf den Einsatz in Textilwaschmittel verändert wurde, und mindestens einen Waschmittelinhaltsstoff, wobei das Textilwaschmittel einen pH-Wert von etwa 9 bis etwa 12 aufweist. Ebenfalls Bestandteil der Erfindung sind die entsprechenden Waschverfahren, die Verwendung der hierin beschriebenen Mittel und die Verwendung von Proteasen in Textilwaschmitteln mit einem pH-Wert von etwa 9 bis etwa 12, sowie die Verwendung von Proteasen in Textilwaschmitteln mit einem pH-Wert von etwa 9 bis etwa 12 zur Entfernung von proteasesensitiven Anschmutzungen auf Textilien.

Der Einsatz von Enzymen in Waschmitteln ist seit Jahrzehnten im Stand der Technik etabliert. Sie dienen dazu, das Leistungsspektrum der betreffenden Mittel entsprechend ihren speziellen Aktivitäten zu erweitern. Hierzu gehören insbesondere hydrolytische Enzyme wie Proteasen, Amylasen, Lipasen und Cellulasen. Die ersten drei genannten hydrolysieren Proteine, Stärke und Fette und tragen somit unmittelbar zur Schmutzentfernung bei. Cellulasen werden insbesondere wegen ihrer Gewebewirkung eingesetzt. Eine weitere Gruppe von Waschmittelenzymen sind oxidative Enzyme, insbesondere Oxidasen, die ggf. im Zusammenspiel mit anderen Komponenten vorzugsweise dazu dienen, Anschmutzungen zu bleichen oder die bleichenden Agentien *in situ zu* erzeugen. Neben diesen Enzymen, die einer fortwährenden Optimierung unterworfen werden, werden laufend weitere Enzyme für den Einsatz in Waschmitteln bereitgestellt, um insbesondere spezielle Anschmutzungen optimal angehen zu können, wie z.B. Pektinasen, β-Glucanasen, Mannanasen oder weitere Hemicellulasen (Glykosidasen) zur Hydrolyse insbesondere spezieller pflanzlicher Polymere.

Die am längsten etablierten und in praktisch allen modernen, leistungsfähigen Waschmitteln enthaltenen Enzyme sind Proteasen. Sie gehören damit zu den technisch bedeutendsten Enzymen überhaupt. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im alkalischen Bereich bei ungefähr pH 9. Einen Überblick über diese Familie bietet z.B. der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von *Bacillus-Spezies* gebildeten und sezernierten Subtilisine als bedeutendste Gruppe innerhalb der Subtilasen zu erwähnen.

Beispiele für die in Waschmitteln bevorzugt eingesetzten Proteasen vom Subtilisin-Typ sind die Subtilisine BPN' und Carlsberg, die Protease PB92, die Subtilisine 147 und 309, die Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen.

In der europäischen Patentanmeldung EP 2016175 A1 ist beispielsweise eine für Wasch- und Reinigungsmittel vorgesehene Protease aus *Bacillus pumilus* offenbart. Generell sind nur ausgewählte Proteasen für den Einsatz in flüssigen tensidhaltigen Zubereitungen überhaupt geeignet. Viele Proteasen zeigen in derartigen Zubereitungen keine ausreichende katalytische Leistung oder aber sie sind nicht ausreichend stabil. Für die Anwendung von Proteasen in Textilwaschmitteln ist daher eine hohe katalytische Aktivität und Stabilität unter Bedingungen, wie sie sich während eines Waschvorgangs darstellen, besonders wünschenswert.

Feste Textilwaschmittel haben in der Regel einen pH-Wert von etwa 8 bis etwa 10, während flüssige Textilwaschmittel einen pH-Wert von etwa 7 bis etwa 9 aufweisen können. Alkalische flüssige Waschmittel sind relativ weit verbreitet und erfreuen sich insbesondere in Nordamerika großer Beliebtheit. Durch den alkalischen pH-Wert ist es jedoch schwierig, geeignete Enzyme zu finden und diese in alkalische flüssige Waschmittel einzuformulieren, da der hohe pH-Wert nicht nur die Leistung der Enzyme, sondern auch ihre Lagerstabilität negativ beeinflussen kann. Zudem sind Enzyme generell in flüssigen Waschmitteln weniger stabil, wenn die Waschmittel einen hohen Wassergehalt aufweisen.

Folglich haben protease- und tensidhaltige flüssige Formulierungen aus dem Stand der Technik den Nachteil, dass die enthaltenen Proteasen unter Standard-Waschbedingungen (z.B. in einem Temperaturbereich von 20 bis 40°C), insbesondere bei einem höheren pH-Wert, keine zufriedenstellende proteolytische Aktivität aufweisen oder nicht ausreichend lagerstabil sind, und die Formulierungen daher keine optimale Reinigungsleistung an proteasesensitiven Anschmutzungen zeigen.

Ein Ziel bei der Entwicklung von Waschmittelrezepturen besteht daher darin, die enthaltenen Enzyme durch aus dem Stand der Technik bekannte Verfahren gezielt oder zufallsbasiert zu verändern und so für den Einsatz in Waschmitteln zu optimieren. Dazu gehören z.B. Punktmutagenese, Deletions- oder Insertionsmutagenese oder Fusion mit anderen Proteinen oder Proteinteilen.

Obwohl aus dem Stand der Technik eine Reihe von Proteasen für Waschmittel bekannt sind, besteht nachwievor ein Bedarf an Proteasen für die Anwendung in hochalkalischen Textilwaschmitteln, insbesondere flüssigen Textilwaschmitteln mit einem pH-Wert von etwa 9 bis etwa 12.

Es bestand daher die Aufgabe, die Stabilität von Proteasen in hochalkalischen Textilwaschmitteln und somit die Reinigungsleistung von Textilwaschmitteln weiter zu verbessern. Überraschenderweise wurde nun festgestellt, dass eine Protease, wie hierin definiert, oder eine hierzu hinreichend ähnliche Protease (bezogen auf die Sequenzidentität) gegenüber herkömmlichen Proteasen und/oder gegenüber der Wildtypform (SEQ ID NO:1) hinsichtlich ihrer Lagerstabilität verbessert ist und daher besonders für den Einsatz in Textilwaschmitteln mit einem pH-Wert von etwa 9 bis etwa 12 geeignet ist.

Gegenstand der vorliegenden Erfindung ist daher in einem ersten Aspekt ein Textilwaschmittel, umfassend a) mindestens eine Protease, wobei die Protease proteolytische Aktivität aufweist und eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an den Positionen, die den Positionen 9, 130, 133, 144, 217, 252 und 271 entsprechen, Aminosäuresubstitutionen, insbesondere die Aminosäuresubstitutionen 9T, 130D, 130V, 133A, 144K, 217M, 252T und 271E, und (ii) an mindestens einer, vorzugsweise mindestens zwei, der Positionen, die den Positionen 6, 89, 131, 166, 189, 211 oder 224 entsprechen, mindestens eine weitere Aminosäuresubstitution, insbesondere ausgewählt aus 6W, 6F, 89A, 89G, 131H, 131Y, 131F, 166M, 166L, 166I, 189T, 189L, 189I, 211N, 211Q, 224A und 224G, bevorzugter ausgewählt aus 6W, 89A, 131H, 166M, 189T, 211N und 224A, aufweist; und b) mindestens einen Waschmittelinhaltsstoff, vorzugsweise in einer Menge von 0,01 bis 99,9 Gew.-%, wobei das Textilwaschmittel einen pH-Wert von etwa 9 bis etwa 12, gemessen in 1 Gew.-%-iger Lösung in entionisiertem Wasser bei 20°C, aufweist. Bevorzugt ist das erfindungsgemäße Waschmittel ein flüssiges Textilwaschmittel. Weiter bevorzugt weist das erfindungsgemäße Textilwaschmittel in 1 Gew.-%-iger Lösung in entionisiertem Wasser bei 20°C einen pH-Wert in einem Bereich von 9 bis 12, insbesondere 9,5 bis 11,5, weiter bevorzugt von 10 bis 11, besonders bevorzugt pH 10 auf.

WO2019/048495 lehrt Proteasevarianten aus *B. pumilus,* insbesondere eine Variante mit den Mutationen 9T, 130D, 133A, 144K, 217M, 252T und 271E (D5, Mutante 38). Der Gegenstand der vorliegenden Anmeldung unterscheidet sich von der Lehre der WO2019/048495 durch die unter (ii) genannten Mutationen und den pH-Wert (vorliegende Anmeldung: pH 9-12; WO2019/048495: im Beispiel kein pH genannt). Die zusätzlichen Mutationen an den Positionen 6, 89, 131, 166, 189, 211 oder 224 (vorzugsweise ausgewählt aus 6W/F, 89A/G, 131H/Y/F, 166M/L/I, 189T/L/I, 211N/Q und 224A/G) bewirken eine verbesserte Lagerstabilität bei hohem pH (siehe Seite 3, Abs. 1-4 der ursprünglich eingereichten Beschreibung; Beispiel).

Erfindungsgemäße Textilwaschmittel zeigen insbesondere auch dann Leistungsvorteile gegenüber anderen Textilwaschmitteln, wenn die Textilwaschmittel mindestens ein zusätzliches Enzym derselben oder einer anderen Art, also z.B. Amylase, Cellulase, Lipase, Mannanase oder Pektinase enthalten, wobei die Auflistung der weiteren Enzyme nicht vollständig ist. Daher ist es bevorzugt, dass die erfindungsgemäßen Textilwaschmittel mindestens ein zusätzliches Enzym derselben Art (also eine weitere Protease) oder einer anderen Art enthalten. Vorzugsweise umfasst das Waschmittel mindestens eine Amylase, wobei die Amylase amylolytische Aktivität aufweist und ausgewählt ist aus a) einer α-Amylase, die eine Aminosäuresequenz umfasst, die zu der in SEQ ID NO:2 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO:2 aufweist, vorzugsweise ausgewählt aus der aus M202L, M202V, M202S, M202T, M202I, M202Q, M202W, S255N, R172Q und Kombinationen davon bestehenden Gruppe; und/oder b) einer α-Amylase, die eine Aminosäuresequenz umfasst, die zu der in SEQ ID NO:3 angegebenen Aminosäuresequenz über deren Gesamtlänge zu mindestens 60% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:3 aufweist, vorzugsweise mindestens eine Aminosäuresubstitution an einer der Positionen 9, 26, 149, 182, 186, 202, 257, 295, 299, 323, 339 and 345, und/oder besonders bevorzugt mindestens eine Aminosäuresubstitution oder -deletion, die aus der aus R118K, D183*, G184*, N195F, R320K, R458K und Kombinationen davon bestehenden Gruppe ausgewählt ist; und/oder c) einer α-Amylase, die eine Aminosäuresequenz umfasst, die zu der in SEQ ID NO:4 angegebenen Aminosäuresequenz über deren Gesamtlänge zu mindestens 90% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:4, aufweist, vorzugsweise Aminosäuredeletionen an den Positionen 183 und 184. Solche Amylasen sind aus z.B. WO2013/063460 und EP2357220 bekannt.

Ein weiterer Gegenstand der Erfindung ist ein Verfahren zur Herstellung eines solchen Textilwaschmittels sowie ein Verfahren zur Reinigung von Textilien, in dem ein erfindungsgemäßes Waschmittel angewendet wird.

Ein weiterer Gegenstand der Erfindung ist eine Verwendung eines solchen Textilwaschmittels zur Reinigung von Textilien.

Ein weiterer Gegenstand der Erfindung ist eine Verwendung einer Protease, die eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an den Positionen, die den Positionen 9, 130, 133, 144, 217, 252 und 271 entsprechen, Aminosäuresubstitutionen, insbesondere die Aminosäuresubstitutionen 9T, 130D, 130V, 133A, 144K, 217M, 252T und 271E, und (ii) an mindestens einer, vorzugsweise mindestens zwei, der Positionen, die den Positionen 6, 89, 131, 166, 189, 211 oder 224 entsprechen, mindestens eine weitere Aminosäuresubstitution, insbesondere ausgewählt aus 6W, 6F, 89A, 89G, 131H, 131Y, 131F, 166M, 166L, 166I, 189T, 189L, 1891, 211N, 211Q, 224A und 224G, bevorzugter ausgewählt aus 6W, 89A, 131H, 166M, 189T, 211N und 224A, aufweist, in einem Textilwaschmittel, vorzugsweise einem flüssigen Textilwaschmittel, zur Entfernung von proteasesensitiven Anschmutzungen von Textilien, wobei das Textilwaschmittel einen pH-Wert von etwa 9 bis etwa 12, gemessen in 1 Gew.-%-iger Lösung in entionisiertem Wasser bei 20°C aufweist.

Besonders bevorzugt wird in den erfindungsgemäßen Mitteln, Verfahren und Verwendungen eine Protease eingesetzt, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an den Positionen, die den Positionen 9, 130, 133, 144, 217, 252 und 271 entsprechen, die Aminosäuresubstitutionen P9T, N130D, T133A, N144K, Y217M, N252T und Q271E, und (ii) an mindestens zwei der Positionen, die den Positionen 6, 89, 131, 166, 189, 211 oder 224 entsprechen, mindestens zwei weitere Aminosäuresubstitutionen, die aus der aus Y6W, Y6F, S89A, S89G, G131H, G131Y, G131F, G166M, G166L, G166I, S189T, S189L, S189I, S211N, S211Q, S224A und S224G, vorzugsweise Y6W, S89A, G131H, G166M, S189T, S211N und S224A, bestehenden Gruppe ausgewählt sind, aufweist.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichtsprozent (Gew.-%).

Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens eine", wie hierin verwendet, bedeutet eine oder mehrere, d.h. 1, 2, 3, 4, 5, 6, 7, 8, 9, 10, 11, 12, 13, 14 oder mehr.

Der Begriff "Waschmittel", wie hierin verwendet, ist gleichbedeutend mit dem Begriff "Textilwaschmittel" oder "Mittel" und bezeichnet eine Zusammensetzung zum Reinigen von Textilien wie in der Beschreibung erläutert.

"Etwa", "ca." oder "ungefähr", wie hierin in Bezug auf einen Zahlenwert verwendet, beziehen sich auf den entsprechenden Zahlenwert ±10%, vorzugsweise ±5%.

"Flüssig", wie hierin verwendet, schließt Flüssigkeiten und Gele sowie auch pastöse Zusammensetzungen ein. Es ist bevorzugt, dass die flüssigen Zusammensetzungen bei Raumtemperatur fließfähig und gießbar sind, es ist aber auch möglich, dass sie eine Fließgrenze aufweisen.

"Hochalkalisch", wie hierin verwendet, meint einen pH-Wert von etwa 9 bis etwa 12, gemessen in 1 Gew.-%-iger Lösung in entionisiertem Wasser bei 20°C.

Die vorliegende Erfindung basiert auf der überraschenden Erkenntnis der Erfinder, dass Aminosäuresubstitutionen an den hierin beschriebenen Positionen eine gegenüber herkömmlichen Proteasen und/oder gegenüber dem Wildtyp verbesserte Lagerstabilität dieser veränderten Protease in hochalkalischen Waschmitteln bewirkt.

Das ist insbesondere insoweit überraschend, als bisher keine solchen Proteasen bzw. keine solcher Aminosäuresubstitutionen mit einer verbesserten Lagerstabilität der entsprechenden Protease in hochalkalischen Waschmitteln, insbesondere flüssigen hochalkalischen Waschmitteln, in Verbindung gebracht wurden.

In bevorzugten Ausführungsformen führt/führen die erfindungsgemäße Veränderung(en) der erfindungsgemäß eingesetzten Proteasen, jeweils bezogen auf die Nummerierung gemäß SEQ ID NO:1, (i) an den Positionen, die den Positionen 9, 130, 133, 144, 217, 252 und 271 entsprechen, und (ii) an mindestens einer der Positionen, die den Positionen 6, 89, 131, 166, 189, 211 oder 224 entsprechen, zu einer verbesserten Lagerstabilität dieser veränderten Protease in hochalkalischen Textilwaschmitteln (pH 9 bis 12).

In weiter bevorzugten Ausführungsformen der erfindungsgemäß eingesetzten Proteasen weist die Protease, jeweils bezogen auf die Nummerierung gemäß SEQ ID NO:1, (i) an den Positionen, die den Positionen 9, 130, 133, 144, 217, 252 und 271 entsprechen, die Aminosäuresubstitutionen P9T, N130D, T133A, N144K, Y217M, N252T und Q271E und (ii) an mindestens einer der Positionen, die den Positionen 6, 89, 131, 166, 189, 211 oder 224 entsprechen, mindestens eine Aminosäuresubstitution, die aus der aus Y6W, Y6F, S89A, S89G, G131H, G131Y, G131F, S166M, S166L, S166I, S189T, S189L, S189I, S211N, S211Q, S224A und S224G bestehenden Gruppe ausgewählt ist, auf.

In bevorzugten Ausführungsformen weist die erfindungsgemäß eingesetzte Protease, jeweils bezogen auf die Nummerierung gemäß SEQ ID NO:1, (i) an den Positionen, die den Positionen 9, 130, 133, 144, 217, 252 und 271 entsprechen, die Aminosäuresubstitutionen P9T, N130D, T133A, N144K, Y217M, N252T und Q271E und (ii) an mindestens einer der Positionen, die den Positionen 6, 89, 131, 166, 189, 211 oder 224 entsprechen, mindestens eine Aminosäuresubstitution, die aus der aus Y6W, Y6F, S89A, S89G, G131H, G131Y, G131F, S166M, S166L, S166I, S189T, S189L, S189I, S211N, S211Q, S224A und S224G bestehenden Gruppe ausgewählt ist, auf, wobei die Kombination der Aminosäuresubstitutionen aus Gruppe (i) und der mindestens einen Aminosäuresubstitution aus Gruppe (ii) zu einer verbesserten Lagerstabilität dieser veränderten Protease in hochalkalischen Textilwaschmitteln (pH 9 bis 12) führt.

In besonders bevorzugten Ausführungsformen enthält das erfindungsgemäße Waschmittel eine Protease mit einer der folgenden Aminosäuresubstitutionsvarianten:
(i) P9T+N130D+T133A+N144K+G166M+S189T+Y217M+N252T+Q271E;
(ii) P9T+N130D+T133A+N144K+G166M+S189T+Y217M+S224A+N252T+Q271E;
(iii) P9T+S89A+N130D+G131H+T133A+N144K+S189T+Y217M+S224A+N252T+Q271E;
(iv) Y6W+P9T+N130D+T133A+N144K+S189T+Y217M+S224A+N252T+Q271E;
(v) P9T+N130D+T133A+N144K+G166M+S211N+Y217M+N252T+Q271E;
(vi) P9T+S89A+N130D+T133A+N144K+S189T+Y217M+S224A+N252T+Q271E,
wobei die Nummerierung jeweils bezogen ist auf die Nummerierung gemäß SEQ ID NO:1.

Bestimmte Ausführungsformen der erfindungsgemäß eingesetzten Proteasen verfügen über eine verbesserte Lagerstabilität. Sie verfügen über eine erhöhte Stabilität in hochalkalischen Textilwaschmitteln im Vergleich zu herkömmlichen Proteasen und/oder zu dem Wildtypenzym (SEQ ID NO:1), insbesondere bei einer Lagerung von 3 oder mehr Tagen, 4 oder mehr Tagen, 7 oder mehr Tagen, 10 oder mehr Tagen, 12 oder mehr Tagen, 14 oder mehr Tagen, 21 oder mehr Tagen oder 28 oder mehr Tagen, wobei das Textilwaschmittel einen pH-Wert von etwa 9 bis etwa 12, gemessen in 1 Gew.-%-iger Lösung in entionisiertem Wasser bei 20°C, aufweist.

Bestimmte Ausführungsformen der erfindungsgemäßen Proteasen können unabhängig von oder zusätzlich zu der erhöhten Lagerstabilität über eine erhöhte katalytische Aktivität in hochalkalischen Textilwaschmitteln verfügen. In vielfältigen Ausführungsformen können die erfindungsgemäß eingesetzten Proteasen eine proteolytische Aktivität besitzen, die bezogen auf den Wildtyp (SEQ ID NO:1) mindestens 101%, 102%, 103%, 104%, 105%, 106%, 107%, 108%, 109% oder 110% beträgt. Solche leistungsverbesserten Proteasen ermöglichen verbesserte Waschergebnisse an proteasesensitiven Anschmutzungen in verschiedenen Temperaturbereichen, insbesondere einem Temperaturbereich von 20 bis 40°C.

Ferner verfügen bevorzugte Ausführungsformen erfindungsgemäß eingesetzter Proteasen über eine besondere Stabilität in Waschmitteln, beispielsweise gegenüber Tensiden und/oder Bleichmitteln und/oder Chelatoren, und/oder gegenüber Temperatureinflüssen, insbesondere gegenüber hohen Temperaturen, beispielsweise zwischen 50 und 65°C, insbesondere 60°C, und/oder gegenüber pH-Wert-Änderungen und/oder gegenüber denaturierenden oder oxidierenden Agentien und/oder gegenüber proteolytischem Abbau und/oder gegenüber einer Veränderung der Redox-Verhältnisse. Mit besonders bevorzugten Ausführungsformen der Erfindung werden folglich leistungsverbesserte und/oder temperaturstabilere Proteasevarianten bereitgestellt. Mit weiteren besonders bevorzugten Ausführungsformen der Erfindung werden leistungsverbesserte und temperaturstabilere Proteasevarianten bereitgestellt. Solche vorteilhaften Ausführungsformen erfindungsgemäßer Proteasen ermöglichen folglich verbesserte Waschergebnisse an proteasesensitiven Anschmutzungen in einem weiten Temperaturbereich.

Die erfindungsgemäß eingesetzten Proteasen weisen enzymatische Aktivität auf, d.h. sie sind zur Hydrolyse von Peptiden und Proteinen befähigt, insbesondere in Waschmittel. Eine erfindungsgemäß eingesetzte Protease ist daher ein Enzym, welches die Hydrolyse von Amid/Peptidbindungen in Protein/Peptid-Substraten katalysiert und dadurch in der Lage ist, Proteine oder Peptide zu spalten. Ferner handelt es sich bei einer erfindungsgemäß eingesetzten Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die erfindungsgemäß eingesetzte Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Proteasemolekül(e) durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von *Bacillus pumilus* oder *Bacillus gibsonii* oder *Bacillus subtilis,* die die erfindungsgemäßen Proteasen exprimieren, oder Zellkulturüberstände solcher Zellen enthalten.

In bevorzugten Ausführungsformen der Erfindung umfasst die erfindungsgemäß eingesetzte Protease eine Aminosäuresequenz, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5% und 97% identisch ist, und jeweils bezogen auf die Nummerierung gemäß SEQ ID NO:1 (i) die Aminosäuresubstitutionen P9T, N130D, T133A, N144K, Y217M, N252T und Q271E aufweist, und (ii) mindestens eine Aminosäuresubstitution an mindestens einer der Positionen, die den Positionen 6, 89, 131, 166, 189, 211 oder 224 entsprechen, aufweist, wobei die mindestens eine Aminosäuresubstitution aus der aus Y6W, Y6F, S89A, S89G, G131 H, G131Y, G131F, S166M, S166L, S166I, S189T, S189L, S189I, S211N, S211Q, S224A und S224G bestehenden Gruppe ausgewählt ist.

In bevorzugten Ausführungsformen der Erfindung umfasst die erfindungsgemäß eingesetzte Protease eine Aminosäuresequenz, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96% und 96,5% identisch ist, und jeweils bezogen auf die Nummerierung gemäß SEQ ID NO:1 (i) die Aminosäuresubstitutionen P9T, N130D, T133A, N144K, Y217M, N252T und Q271E aufweist, und (ii) mindestens zwei Aminosäuresubstitutionen an mindestens zwei der Positionen, die den Positionen 6, 89, 131, 166, 189, 211 oder 224 entsprechen, aufweist, wobei die mindestens zwei Aminosäuresubstitutionen aus der aus Y6W, Y6F, S89A, S89G, G131H, G131Y, G131F, S166M, S166L, S166I, S189T, S189L, S189I, S211N, S211Q, S224A und S224G bestehenden Gruppe ausgewählt sind.

In bevorzugten Ausführungsformen der Erfindung umfasst die erfindungsgemäß eingesetzte Protease eine Aminosäuresequenz, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5% und 96% identisch ist, und jeweils bezogen auf die Nummerierung gemäß SEQ ID NO:1 (i) die Aminosäuresubstitutionen P9T, N130D, T133A, N144K, Y217M, N252T und Q271E aufweist, und (ii) mindestens drei Aminosäuresubstitutionen an mindestens drei der Positionen, die den Positionen 6, 89, 131, 166, 189, 211 oder 224 entsprechen, aufweist, wobei die mindestens drei Aminosäuresubstitutionen aus der aus Y6W, Y6F, S89A, S89G, G131H, G131Y, G131F, S166M, S166L, S166I, S189T, S189L, S189I, S211N, S211Q, S224A und S224G bestehenden Gruppe ausgewählt sind.

In bevorzugten Ausführungsformen der Erfindung umfasst die erfindungsgemäß eingesetzte Protease eine Aminosäuresequenz, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5% und 96% identisch ist, und jeweils bezogen auf die Nummerierung gemäß SEQ ID NO:1 (i) die Aminosäuresubstitutionen P9T, N130D, T133A, N144K, Y217M, N252T und Q271E aufweist, und (ii) mindestens vier Aminosäuresubstitutionen an mindestens vier der Positionen, die den Positionen 6, 89, 131, 166, 189, 211 oder 224 entsprechen, aufweist, wobei die mindestens vier Aminosäuresubstitutionen aus der aus Y6W, Y6F, S89A, S89G, G131H, G131Y, G131F, S166M, S166L, S166I, S189T, S189L, S189I, S211N, S211Q, S224A und S224G bestehenden Gruppe ausgewählt sind.

In besonders bevorzugten Ausführungsformen enthält die erfindungsgemäß eingesetzte Protease eine der folgenden Aminosäuresubstitutionsvarianten:
(i) P9T+N130D+T133A+N144K+G166M+S189T+Y217M+N252T+Q271E;
(ii) P9T+N130D+T133A+N144K+G166M+S189T+Y217M+S224A+N252T+Q271E;
(iii) P9T+S89A+N130D+G131H+T133A+N144K+S189T+Y217M+S224A+N252T+Q271E;
(iv) Y6W+P9T+N130D+T133A+N144K+S189T+Y217M+S224A+N252T+Q271E;
(v) P9T+N130D+T133A+N144K+G166M+S211N+Y217M+N252T+Q271E;
(vi) P9T+S89A+N130D+T133A+N144K+S189T+Y217M+S224A+N252T+Q271E,
wobei die Nummerierung jeweils auf die Nummerierung gemäß SEQ ID NO:1 bezogen ist.

Im Zusammenhang mit der vorliegenden Erfindung bedeutet das Merkmal, dass eine Protease mindestens eine der angegebenen Aminosäuresubstitutionen aufweist, dass sie an der jeweiligen Position eine (der angegebenen) Aminosäuresubstitution(en) enthält, d.h. zumindest die angegebenen Positionen nicht anderweitig mutiert oder, beispielsweise durch Fragmentierung der Protease, deletiert sind.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) "Basic local alignment search tool", J. Mol. Biol. 215:403-410 und Altschul et al. (1997) "Gapped BLAST and PSI-BLAST: a new generation of protein database search programs", Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden z.B. die Clustal-Serie (vgl. z.B. Chenna et al. (2003) "Multiple sequence alignment with the Clustal series of programs", Nucleic Acid Res. 31:3497-3500), T-Coffee (vgl. z.B. Notredame et al. (2000) "T-Coffee: A novel method for multiple sequence alignments", J. Mol. Biol. 302:205-217) oder Programme, die auf diesen Programmen bzw. Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, d.h. dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäureaustausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt A95G die Substitution von Alanin an Position 95 durch Glycin, A95AG die Insertion von Glycin nach der Aminosäure Alanin an Position 95 und A95* oder ΔA59 die Deletion von Alanin an Position 95. Diese Nomenklatur ist dem Fachmann auf dem Gebiet der Enzymtechnologie bekannt.

Ein weiterer Gegenstand der vorliegenden Erfindung ist ein Waschmittel mit einer Protease, dadurch gekennzeichnet, dass die Protease aus einer erfindungsgemäßen Protease als Ausgangsmolekül durch ein- oder mehrfache konservative Aminosäuresubstitution erhältlich ist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z.B. der Austausch eines unpolaren Aminosäurerestes gegen einen anderen unpolaren Aminosäurerest. Konservative Aminosäuresubstitutionen im Rahmen der Erfindung umfassen z.B.: G=A=S, I=V=L=M, D=E, N=Q, K=R, Y=F, S=T, G=A=I=V=L=M=Y=F=W=P=S=T.

Ein weiterer Gegenstand der vorliegenden Erfindung ist ein Waschmittel, umfassend eine Protease, dadurch gekennzeichnet ist, dass die Protease aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 260, 270, 271, 272, 273, 274 oder 275 zusammenhängenden Aminosäuren mit dem Ausgangsmolekül übereinstimmt, wobei die Protease (i) an den Positionen, die den Positionen 9, 130, 133, 144, 217, 252 oder 271 entsprechen, und (ii) mindestens eine Aminosäuresubstitution an mindestens einer der Positionen, die den Positionen 6, 89, 131, 166, 189, 211 oder 224 entsprechen, aufweist.

So ist es z.B. möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese z.B. auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 80%, vorzugsweise mindestens 90% der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Die Aminosäurepositionen werden hierbei durch ein Alignment der Aminosäuresequenz einer erfindungsgemäßen Protease mit der Aminosäuresequenz der Protease aus *Bacillus pumilus,* wie sie in SEQ ID NO:1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere oder niedrigere Zahl von Aminosäureresten umfasst als die Protease aus *Bacillus pumilus* gemäß SEQ ID NO:1. Ausgehend von den genannten Positionen in der Aminosäuresequenz der Protease aus *Bacillus pumilus* sind die Veränderungspositionen in einer erfindungsgemäßen Protease diejenigen, die eben diesen Positionen in einem Alignment zugeordnet sind.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht z.B. ein Aminosäureaustausch in einer bestimmten Position der Protease gemäß SEQ ID NO:1 mit einer Veränderung eines enzymatischen Parameters einher, beispielsweise mit der Erhöhung des K_{M}-Wertes, und wird eine entsprechende Veränderung des enzymatischen Parameters, z.B. also ebenfalls eine Erhöhung des K_{M}-Wertes, in einer erfindungsgemäßen Proteasevariante beobachtet, deren Aminosäureaustausch durch dieselbe eingeführte Aminosäure erreicht wurde, so ist hierin eine Bestätigung der korrekten Zuordnung zu sehen.

Eine erfindungsgemäße Protease kann zusätzlich stabilisiert sein, insbesondere durch eine oder mehrere Mutationen, z.B. Substitutionen, oder durch Kopplung an ein Polymer. Denn eine Erhöhung der Stabilität bei der Lagerung und/oder während des Einsatzes, z.B. beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Weitere Möglichkeiten der Stabilisierung sind z.B.:
- Veränderung der Bindung von Metallionen, insbesondere der Calcium-Bindungsstellen, z.B. durch Austauschen einer oder mehrerer der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss denaturierender Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Protease wie vorstehend beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert. Unter Derivaten werden im Sinne der vorliegenden Anmeldung demnach solche Proteine verstanden, deren reine Aminosäurekette chemisch modifiziert worden ist. Solche Derivatisierungen können *z.B. in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das z.B. über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können z.B. die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann z.B. für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit z.B. dessen Hautverträglichkeit zu erhöhen. Beispielsweise können Kopplungen mit makromolekularen Verbindungen, z.B. Polyethylenglykol, das Protein hinsichtlich der Stabilität und/oder Hautverträglichkeit verbessern. Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, z.B. aus der Kultur der produzierenden Mikroorganismen. Ein Protein kann auch, z.B. zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann z.B. über entsprechende Begleitstoffe gesteuert werden. Insbesondere die gemeinsame Präparation von Proteasen mit spezifischen Inhibitoren ist diesbezüglich möglich.

Unter allen vorstehend beschriebenen Proteasen beziehungsweise Proteasevarianten und/oder Derivaten sind im Rahmen der vorliegenden Erfindung diejenigen besonders bevorzugt, deren Lagerstabilität und/oder deren Reinigungsleistung gegenüber der Ausgangsvariante verbessert ist, wobei die Reinigungsleistung in einem Waschsystem bestimmt wird wie weiter unten beschrieben. Zahlreiche Proteasen und insbesondere Subtilisine werden als sogenannte Präproteine, also zusammen mit einem Propeptid und einem Signalpeptid gebildet, wobei die Funktion des Signalpeptids üblicherweise darin besteht, die Ausschleusung der Protease aus der sie produzierenden Zelle in das Periplasma oder das die Zelle umgebende Medium zu gewährleisten, und das Propeptid üblicherweise für die korrekte Faltung der Protease nötig ist. Das Signalpeptid und das Propeptid sind in der Regel der N-terminale Teil des Präproteins. Das Signalpeptid wird unter natürlichen Bedingungen durch eine Signalpeptidase von der übrigen Protease abgespalten. Anschließend erfolgt die durch das Propeptid unterstützte korrekte endgültige Faltung der Protease. Die Protease ist dann in ihrer aktiven Form und spaltet das Propeptid selbst ab. Nach der Abspaltung des Propeptids übt die dann reife (mature) Protease, insbesondere Subtilisin, ihre katalytische Aktivität ohne die ursprünglich vorhandenen N-terminalen Aminosäuren aus. Für technische Anwendungen allgemein und insbesondere im Rahmen der Erfindung sind die reifen (maturen) Proteasen, d.h. die nach ihrer Herstellung prozessierten Enzyme, gegenüber den Präproteinen bevorzugt. Die Proteasen können ferner von den sie produzierenden Zellen nach der Herstellung der Polypeptidkette modifiziert werden, z.B. durch Anknüpfung von Zuckermolekülen, Formylierungen, Aminierungen, usw. Solche Modifikationen sind posttranslationale Modifikationen und können, müssen jedoch nicht einen Einfluss auf die Funktion der Protease ausüben.

"Variante", wie hierin verwendet, bezieht sich auf natürliche oder artifiziell erzeugte Variationen einer nativen Protease, die eine gegenüber der Referenzform abgewandelte Aminosäuresequenz aufweist. Zusätzlich zu den vorstehend erläuterten Aminosäureveränderungen können erfindungsgemäße Proteasen weitere Aminosäureveränderungen, insbesondere Aminosäuresubstitutionen, -insertionen oder -deletionen, aufweisen. Solche Proteasen sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden. Das Ziel ist es, in die bekannten Moleküle gezielte Mutationen wie Substitutionen, Insertionen oder Deletionen einzuführen, um z.B. die Reinigungsleistung von Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität des Enzyms erhöht und dadurch seine Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease kann daher im Rahmen der Erfindung zusätzlich weiterentwickelt sein, z.B. hinsichtlich ihrer Stabilität gegenüber Tensiden und/oder Bleichmitteln und/oder anderen Komponenten.

Ein erfindungsgemäßes Waschmittel enthält die Protease zunehmend bevorzugt in einer Menge von 1 x 10⁻⁸ bis 5 Gew.-%, von 0,0001 bis 1 Gew.-%, von 0,0005 bis 0,5 Gew.-%, von 0,001 bis 0,1 Gew.-%, jeweils bezogen auf aktives Protein und bezogen auf das Gesamtgewicht des Waschmittels.

In einer weiteren Ausführungsform der Erfindung ist die Protease dadurch gekennzeichnet, dass ihre Reinigungsleistung (nach Lagerung, z.B. über 2 Wochen) gegenüber herkömmlichen Proteasen und/oder dem Wildtypenzym (SEQ ID NO:1) nicht signifikant verringert ist, d.h. mindestens 80% der Referenzwaschleistung besitzt, vorzugsweise mindestens 100%, noch bevorzugter mindestens 110% oder mehr.

Unter Reinigungsleistung wird im Rahmen der Erfindung das Vermögen eines Mittels, eine vorhandene Anschmutzung teilweise oder vollständig zu entfernen, insbesondere die Aufhellungsleistung an einer oder mehreren Anschmutzungen auf Textilien, verstanden. Beispiele für solche Anschmutzungen sind Blut auf Baumwolle oder Schokolade-Milch/Ruß auf Baumwolle, Kakao auf Baumwolle oder Porridge auf Baumwolle. Im Rahmen der Erfindung weisen sowohl das Waschmittel, welches die Protease umfasst, bzw. die durch dieses Mittel gebildete Waschflotte, als auch die Protease selbst eine jeweilige Reinigungsleistung auf. Die Reinigungsleistung der Protease trägt somit zur Reinigungsleistung des Mittels bzw. der durch das Mittel gebildeten Waschflotte bei. Die Reinigungsleistung wird bevorzugt ermittelt wie weiter unten angegeben.

Unter Waschflotte wird diejenige das Waschmittel enthaltende Gebrauchslösung verstanden, die auf die Textilien oder Gewebe einwirkt und damit mit den auf den Textilien bzw. Geweben vorhandenen Anschmutzungen in Kontakt kommt. Üblicherweise entsteht die Waschflotte, wenn der Waschvorgang beginnt und das Waschmittel z.B. in einer Waschmaschine oder in einem anderen geeigneten Behältnis mit Wasser verdünnt wird.

Die Reinigungsleistung kann in einem Waschsystem bestimmt werden, das ein Waschmittel in einer Dosierung zwischen 2,0 und 8,0 Gramm pro Liter Waschflotte sowie die Protease enthält. Die zu vergleichenden Proteasen werden konzentrationsgleich (bezogen auf aktives Protein) eingesetzt. Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also z.B. die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Die Konzentration der Protease in dem für dieses Waschsystem bestimmten Waschmittel beträgt 0,0001 bis 0,1 Gew.-%, insbesondere 0,001 bis 0,1 Gew.-%, weiter bevorzugt 0,01 bis 0,06 Gew.-% und besonders bevorzugt 0,001 bis 0,02 Gew.-%, bezogen auf aktives Protein.

Ein bevorzugtes flüssiges Waschmittel für ein solches Waschsystem ist wie folgt zusammengesetzt (alle Angaben in Gewichts-Prozent): <1% Anti-Schaummittel,1-4% Zitronensäure, 0,5-3% Glycerin, 0,3-2% NaOH, 4-8% 1,2-Propandiol, 2-5% FAEOS (Fettalkoholethersulfat), 5-9% nichtionische Tenside (FAEO), 7-12% anionische Tenside (LAS), 0,5-1,5% Proteasestabilisator, 1-3% Palmkernöl-Fettsäuren, 0,5-2% HEDP (1-Hydroxyethan-(1,1-di-phosphonsäure)), 2-6% Monoethanolamin (MEA), 0,2-1% soil release polymer, Rest demineralisiertes Wasser. Bevorzugt beträgt die Dosierung des flüssigen Waschmittels zwischen 2,0 und 8,0 Gramm pro Liter Waschflotte, z.B. 2,5 g/L, 3,2 g/L, 3,5 g/L, 4,0 g/L, 4,7 g/L, 4,9 g/L, 5,5 g/L oder 5,9 g/L Waschflotte bzw. etwa 55 g/Job. Bevorzugt wird in einem pH-Wertebereich von etwa 9 bis etwa 12, bevorzugt von etwa 10 bis 11, besonders bevorzugt bei pH 10, gewaschen.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist z.B. das Spektrometer Minolta CM508d. Üblicherweise werden die für die Messung eingesetzten Geräte zuvor mit einem Weißstandard, bevorzugt einem mitgelieferten Weißstandard, kalibriert.

Dem Fachmann auf dem Gebiet der Enzymtechnologie sind Verfahren zur Bestimmung der Protease-Aktivität geläufig und werden von ihm routinemäßig angewendet. Beispielsweise sind solche Verfahren offenbart in Tenside, Band 7 (1970), S. 125-132. Alternativ kann die Proteaseaktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s. Die Proteaseaktivität wird üblicherweise in Protease-Einheiten (PE) angegeben. Geeignete Protease-Aktivitäten betragen beispielsweise 2,25, 5 oder 10 PE pro ml Waschflotte. Die Proteaseaktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, z.B. dem BCA-Verfahren (Bicinchoninsäure; 2,2'-Bichinolyl-4,4'-dicarbonsäure) oder dem Biuret-Verfahren (Gornall et al. (1948) J. Biol. Chem., 177:751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (Bender et al. (1966) J. Am. Chem. Soc. 88(24):5890-5913) erfolgen.

Bevorzugte Ausführungsformen erfindungsgemäßer Proteasen erzielen solche vorteilhaften Reinigungsleistungen auch schon bei niedrigen Temperaturen, insbesondere in den Temperaturbereichen zwischen 10 und 60°C, bevorzugt zwischen 15 und 50°C und besonders bevorzugt zwischen 20 und 40°C.

Unter einem Waschmittel sind erfindungsgemäß alle denkbaren Waschmittelarten zu verstehen, sowohl Konzentrate als auch unverdünnt anzuwendende Mittel, zum Einsatz im kommerziellen Maßstab, in der Waschmaschine oder bei der Handwäsche bzw. -reinigung. Dazu gehören z.B. Waschmittel für Textilien, Teppiche, oder Naturfasern, für die die Bezeichnung Waschmittel verwendet wird. Zu den Waschmitteln im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Waschmitteln im Rahmen der Erfindung auch Textilvor- und Nachbehandlungsmittel, also solche Mittel, mit denen das Wäschestück vor der eigentlichen Wäsche in Kontakt gebracht wird, z.B. zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Waschmittel, die als pulverförmige oder granulare Feststoffe, in verdichteter oder nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei das Waschmittel einen solchen weiteren Inhaltsstoff vorzugsweise in einer Menge von 0,01 bis 99,9 Gew.-% enthält. Die erfindungsgemäßen Waschmittel können insbesondere Tenside, Builder (Gerüststoffe), Polymere, Glaskorrosionsinhibitoren, Korrosionsinhibitoren, Bleichmittel wie Persauerstoffverbindungen, Bleichaktivatoren oder Bleichkatalysatoren enthalten. Ferner können sie wassermischbare organische Lösungsmittel, weitere Enzyme, Enzymstabilisatoren, Sequestrierungsmittel, Elektrolyte, pH-Regulatoren und/oder weitere Hilfsstoffe wie optische Aufheller, Vergrauungsinhibitoren, Farbübertragungsinhibitoren, Schaumregulatoren sowie Farb- und Duftstoffe sowie Kombinationen hiervon enthalten.

Dabei kommen insbesondere anionische Tenside, nichtionische Tenside und deren Gemische in Frage. Die erfindungsgemäßen Mittel können ein oder mehrere Tenside enthalten, wobei insbesondere anionische Tenside, nichtionische Tenside und deren Gemische in Frage kommen, aber auch kationische, zwitterionische und/oder amphotere Tenside können enthalten sein. Die Mittel enthalten vorzugsweise 5 bis 70 Gew.-% Tensid, bevorzugt 5 bis 55 Gew.-% und weiter bevorzugt 5 bis 35 Gew.-% Tensid. Besonders bevorzugt enthalten die erfindungsgemäßen Wachmittel nicht mehr als 30 Gew.-% Tenside, ganz besonders bevorzugt nicht mehr als 20 Gew.-% Tenside.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten C₁₂₋₁₈-Fettsäuren. Derartige Fettsäuren können auch in nicht vollständig neutralisierter Form eingesetzt werden. Zu den brauchbaren Tensiden des Sulfat-Typs gehören die Salze der Schwefelsäurehalbester von C₁₂₋₁₈-Fettalkoholen und die Sulfatierungsprodukte der genannten nichtionischen Tenside mit niedrigem Ethoxylierungsgrad. Zu den verwendbaren Tensiden vom Sulfonat-Typ gehören z.B. C₉₋₁₄-Alkylbenzolsulfonate, Alkansulfonate, die aus C₁₂₋₁₈-Alkanen z.B. durch Sulfochlorierung oder Sulfoxidation mit anschließender Hydrolyse bzw. Neutralisation gewonnen werden, C₁₂₋₁₈-Olefinsulfonate, die bei der Umsetzung entsprechender Monoolefine mit Schwefeltrioxid entstehen, Gemische aus Alken- und Hydroxyalkansulfonaten, Disulfonaten, wie man sie z.B. aus C₁₂₋₁₈-Monoolefinen mit end- oder innenständigen Doppelbindung durch Sulfonieren mit gasförmigem Schwefeltrioxid und anschließende alkalische oder saure Hydrolyse der Sulfonierungsprodukte erhält, sowie α-Sulfofettsäureester (Estersulfonate), die bei der Sulfonierung von Fettsäuremethyl- oder - ethylestern entstehen, z.B. α-sulfonierte Methylester der hydrierten Kokos-, Palmkern- oder Talgfettsäuren.

Vorzugsweise weist das Mittel 2 bis 55 Gew.-%, vorzugsweise 3 bis 35 Gew.-%, Aniontensid auf. Ganz bevorzugt weist das Mittel 3 bis 15 Gew.-% Alkylbenzolsulfonat auf. Darüber hinaus kann das Mittel vorzugsweise noch weitere anionische Tenside, insbesondere Alkylethersulfate, sowie nichtionische Tenside, insbesondere Fettalkoholalkoxylate, enthalten. Diese können dann den Rest der Tenside ausmachen.

Geeignete Alkylbenzolsulfonate sind vorzugsweise ausgewählt aus linearen oder verzweigten Alkylbenzolsulfonaten der Formel 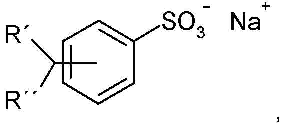 in der R' und R" unabhängig H oder Alkyl sind und zusammen 6 bis 19, vorzugsweise 7 bis 15 und insbesondere 9 bis 13 C-Atome enthalten. Ein ganz besonders bevorzugter Vertreter ist Natriumdodecylbenzylsulfonat.

Als Alk(en)ylsulfate werden die Alkali- und insbesondere die Natriumsalze der Schwefelsäurehalbester der C₁₂₋₁₈-Fettalkohole, z.B. aus Kokosfettalkohol, Talgfettalkohol, Lauryl-, Myristyl-, Cetyl- oder Stearylalkohol oder der C₁₀₋₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂₋₁₆-Alkylsulfate und C₁₂₋₁₅-Alkylsulfate sowie C₁₄₋₁₅-Alkylsulfate bevorzugt.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Geeignete Alkylethersulfate sind z.B. Verbindungen der Formel R¹-O-(AO)ₙ-SO₃⁻ X⁺. In dieser Formel steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂₋₁₈-Fettalkoholen, z.B. von Kokosfettalkohol, Talgfettalkohol, Lauryl-, Myristyl-, Cetyl- oder Stearylalkohol oder von C₁₀₋₂₀-Oxoalkoholen. AO steht für eine Ethylenoxid-(EO) oder Propylenoxid-(PO)-Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X⁺ steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺, ½ Mg²⁺, ½ Ca²⁺, ½ Mn²⁺ und deren Mischungen.

In verschiedenen Ausführungsformen kann das Alkylethersulfat ausgewählt sein aus Fettalkoholethersulfaten der Formel 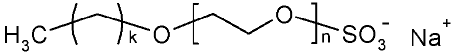 mit k = 11 bis 19, n = 2, 3, 4, 5, 6, 7 oder 8. Ganz besonders bevorzugte Vertreter sind Na-C₁₂₋₁₄-Fettalkoholethersulfate mit 2 EO (k = 11-13, n = 2). Der angegebenen Ethoxylierungsgrad stellt einen statistischen Mittelwert dar, der für ein spezielles Produkt eine ganze oder eine gebrochene Zahl sein kann. Die angegebenen Alkoxylierungsgrade stellen statistische Mittelwerte dar, die für ein spezielles Produkt eine ganze oder eine gebrochene Zahl sein können. Bevorzugte Alkoxylate/Ethoxylate weisen eine eingeengte Homologenverteilung auf (narrow range ethoxylates, NRE).

Es hat sich für die Kaltwaschleistung als vorteilhaft erwiesen, wenn die Waschmittel zusätzlich Seife(n) enthalten. Bevorzugte Waschmittel sind daher dadurch gekennzeichnet, dass sie Seife(n) enthalten. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 8 bis etwa 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören z.B. C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhaft eingesetzt werden kann, sind die Alkylpolyglycoside (APG). Einsetzbare Alkylpolyglycoside genügen der allgemeinen Formel RO(G)z, in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1,0 und 4,0, vorzugsweise zwischen 1,0 und 2,0 und insbesondere zwischen 1,1 und 1,4. Bevorzugt eingesetzt werden lineare Alkylpolyglycoside, also Alkylpolyglycoside, in denen der Polyglycosylrest ein Glucoserest und der Alkylrest ein n-Alkylrest ist.

Auch nichtionische Tenside vom Typ der Aminoxide, z.B. N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

In den erfindungsgemäßen Waschmittel bevorzugt einsetzbare nichtionische Tenside weisen die Formel R¹-CH(OH)CH₂O-(AO)_{w}-(A'O)ₓ-(A"O)_{y}-(A‴O)_{z}-R² auf, in der R¹ für einen geradkettigen oder verzweigten, gesättigten oder ein- bzw. mehrfach ungesättigten C₆₋₂₄-Alkyl- oder -Alkenylrest steht; R² für einen linearen oder verzweigten Kohlenwasserstoffrest mit 2 bis 26 Kohlenstoffatomen steht; A, A', A" und A‴ unabhängig voneinander für einen Rest aus der Gruppe -CH₂CH₂, -CH₂CH₂-CH₂, - CH₂-CH(CH₃), -CH₂-CH₂-CH₂-CH₂, -CH₂-CH(CH₃)-CH₂-, -CH₂-CH(CH₂-CH₃) stehen; w, x, y und z für Werte zwischen 0,5 und 120 stehen, wobei x, y und/oder z auch 0 sein können, bevorzugt sind. Durch den Zusatz der vorgenannten nichtionischen Tenside der Formel R¹-CH(OH)CH₂O-(AO)_{w}-(A'O)ₓ-(A"O)_{y}-(A‴O)_{z}-R², nachfolgend auch als "Hydroxymischether" bezeichnet, kann die Reinigungsleistung erfindungsgemäßer enzymhaltiger Zubereitungen deutlich verbessert werden und zwar sowohl im Vergleich zu tensidfreien Systemen wie auch im Vergleich zu Systemen, die alternative nichtionischen Tenside, z.B. aus der Gruppe der polyalkoxylierten Fettalkohole enthalten.

Durch den Einsatz dieser nichtionischen Tenside mit einer oder mehreren freien Hydroxylgruppe(n) an einem oder beiden endständigen Alkylreste kann die Stabilität der in den erfindungsgemäßen Waschmittelzubereitungen enthaltenen Enzyme deutlich verbessert werden.

Bevorzugt werden insbesondere solche endgruppenverschlossene poly(oxyalkylierten) Niotenside, die, gemäß der Formel R¹O[CH₂CH₂O]ₓCH₂CH(OH)R², neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei x für Werte zwischen 1 und 90, vorzugsweise für Werte zwischen 30 und 80 und insbesondere für Werte zwischen 30 und 60 steht.

Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht. Zur Gruppe dieser nichtionischen Tenside zählen z.B. die C₂₋₂₆ Fettalkohol-(PO)₁-(EO)₁₅₋₄₀-2-hydroxyalkylether, insbesondere auch die C₈₋₁₀ Fettalkohol-(PO)₁-(EO)₂₂-2-hydroxydecylether.

Besonders bevorzugt werden weiterhin solche endgruppenverschlossene poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R³)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15. Wie vorstehend beschrieben, kann jedes R³ in der obenstehenden Formel unterschiedlich sein, falls x ≥ 2 ist. Hierdurch kann die Alkylenoxideinheit in der eckigen Klammer variiert werden. Steht x z.B. für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, z.B. (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und z.B. eine große Anzahl (EO)-Gruppen, kombiniert mit einer geringen Anzahl (PO)-Gruppen einschließt, oder umgekehrt. Besonders bevorzugte endgruppenverschlossene poly(oxyalkylierte) Alkohole der oben stehenden Formel weisen Werte von k = 1 und j = 1 auf, so dass sich die vorstehende Formel zu R¹O[CH₂CH(R³)O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Als besonders wirkungsvoll haben sich schließlich die nichtionischen Tenside der Formel R¹-CH(OH)CH₂O-(AO)_{w}-R² erwiesen, in der R¹ für einen geradkettigen oder verzweigten, gesättigten oder ein- bzw. mehrfach ungesättigten C₆₋₂₄-Alkyl- oder -Alkenylrest steht; R² für einen linearen oder verzweigten Kohlenwasserstoffrest mit 2 bis 26 Kohlenstoffatomen steht; A für einen Rest aus der Gruppe -CH₂CH₂, -CH₂CH₂-CH₂, -CH₂-CH(CH₃) steht, und w für Werte zwischen 1 und 120, vorzugsweise 10 bis 80, insbesondere 20 bis 40 steht. Zur Gruppe dieser nichtionischen Tenside zählen z.B. die C₄₋₂₂-Fettalkohol-(EO)₁₀₋₈₀-2-hydroxyalkylether, insbesondere auch die C₈₋₁₂-Fettalkohol-(EO)₂₂-2-hydroxydecylether und die C₄₋₂₂-Fettalkohol-(EO)₄₀₋₈₀-2-hydroxyalkylether.

Bevorzugte Waschmittel sind dadurch gekennzeichnet, dass das Waschmittel mindestens ein nichtionisches Tensid, vorzugsweise ein nichtionisches Tensid aus der Gruppe der Hydroxymischether, enthält, wobei der Gewichtsanteil des nichtionischen Tensids am Gesamtgewicht des Waschmittels vorzugsweise 0,2 bis 20 Gew.-%, bevorzugt 1 bis 18 Gew.-%, weiter bevorzugt 2 bis 15 Gew.-% und besonders bevorzugt 5 bis 10 Gew.-% beträgt.

Geeignete Amphotenside sind z.B. Betaine der Formel (Rⁱⁱⁱ)(R^{iv})(R^{v})N⁺CH₂COO⁻, in der Rⁱⁱⁱ einen ggf. durch Heteroatome oder Heteroatomgruppen unterbrochenen Alkylrest mit 8 bis 25, vorzugsweise 10 bis 21 Kohlenstoffatomen und R^{iv} sowie R^{v} gleichartige oder verschiedene Alkylreste mit 1 bis 3 Kohlenstoffatomen bedeuten, insbesondere C₁₀₋₁₈-Alkyldimethylcarboxymethylbetain und C₁₁₋₁₇-Alkylamidopropyldimethylcarboxymethylbetain. Geeignete kationische Tenside sind u.a. die quartären Ammoniumverbindungen der Formel (R^{vi})(R^{vii})(R^{viii})(R^{ix})N⁺X⁻, in der R^{vi} bis R^{ix} für vier gleich- oder verschiedenartige, insbesondere zwei lang- und zwei kurzkettige, Alkylreste und X⁻ für ein Anion, insbesondere ein Halogenidion, stehen, z.B. Didecyldimethylammoniumchlorid, Alkylbenzyldidecylammoniumchlorid und deren Mischungen. Weitere geeignete kationische Tenside sind die quaternären oberflächenaktiven Verbindungen, insbesondere mit einer Sulfonium-, Phosphonium-, Jodonium- oder Arsoniumgruppe, die auch als antimikrobielle Wirkstoffe bekannt sind. Durch den Einsatz von quaternären oberflächenaktiven Verbindungen mit antimikrobieller Wirkung kann das Mittel mit einer antimikrobiellen Wirkung ausgestaltet werden bzw. dessen ggf. aufgrund anderer Inhaltsstoffe bereits vorhandene antimikrobielle Wirkung verbessert werden.

Ein weiterer bevorzugter Bestandteil erfindungsgemäßer Waschmittel sind Komplexbildner. Besonders bevorzugte Komplexbildner sind die Phosphonate, sofern ihr Einsatz regulatorisch zulässig ist. Die komplexbildenden Phosphonate umfassen neben der 1-Hydroxyethan-1,1-diphosphonsäure eine Reihe unterschiedlicher Verbindungen, wie z.B. Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Ein im Rahmen dieser Anmeldung bevorzugtes Waschmittel enthält ein oder mehrere Phosphonat(e) aus der Gruppe Aminotrimethylenphosphonsäure (ATMP) und/oder deren Salze; Ethylendiamintetra(methylenphosphonsäure) (EDTMP) und/oder deren Salze; Diethylentriaminpenta(methylenphosphonsäure) (DTPMP) und/oder deren Salze; 1-Hydroxyethan-1,1-diphosphonsäure (HEDP) und/oder deren Salze; 2-Phosphonobutan-1,2,4-tricarbonsäure (PBTC) und/oder deren Salze; Hexamethylendiamintetra(methylenphosphonsäure) (HDTMP) und/oder deren Salze; Nitrilotri(methylenphosphonsäure) (NTMP) und/oder deren Salze. Besonders bevorzugt sind Waschmittel, welche als Phosphonate 1-Hydroxyethan-1,1-diphosphonsäure (HEDP) oder Diethylentriaminpenta(methylenphosphonsäure) (DTPMP) enthalten. Selbstverständlich können die erfindungsgemäßen Waschmittel zwei oder mehr unterschiedliche Phosphonate enthalten. Bevorzugte erfindungsgemäße Waschmittel sind dadurch gekennzeichnet, dass das Waschmittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht des Waschmittels vorzugsweise 0,1 und 8,0 Gew.-%, bevorzugt 0,2 und 5,0 Gew.-%, weiter bevorzugt 0,3 und 3,0 Gew.-% und besonders bevorzugt 0,5-2,0 Gew.-% beträgt.

Die erfindungsgemäßen Waschmittel enthalten weiterhin vorzugsweise Gerüststoff (Builder), vorzugsweise mindestens einen wasserlöslichen und/oder wasserunlöslichen, organischen und/oder anorganischen Builder. Zu den Gerüststoffe zählen dabei insbesondere die Silikate, Carbonate und organische Cobuilder.

Als organische Cobuilder sind insbesondere Polycarboxylate/Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben. Organische Cobuildersubstanzen können gewünschtenfalls in Mengen bis zu 40 Gew.-%, insbesondere bis zu 25 Gew.-% und vorzugsweise von 1 bis 8 Gew.-% enthalten sein.

Brauchbare organische Gerüstsubstanzen sind z.B. die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren und Carboxymethylinuline, monomere und polymere Aminopolycarbonsäuren, insbesondere Glycindiessigsäure, Methylglycindiessigsäure, Glutamindiessigsäure, Nitrilotriessigsäure (NTA), Iminodisuccinat wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendiamintetrakis(methylenphosphonsäure), Lysintetra(methylenphosphosäure) und 1-Hydroxyethan-1,1-diphosphonsäure, polymere Hydroxyverbindungen wie Dextrin sowie polymere (Poly)carbonsäuren, insbesondere durch Oxidation von Polysacchariden bzw. Dextrinen zugängliche Polycarboxylate, und/oder polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Derartige organische Buildersubstanzen können gewünschtenfalls in Mengen bis zu 50 Gew.-%, insbesondere bis zu 25 Gew.-%, vorzugsweise von 10 bis 20 Gew.-% und besonders bevorzugt von 1 bis 5 Gew.-% enthalten sein.

Die freien Säuren besitzen neben ihrer Builderwirkung typischerweise auch die Eigenschaft einer Säuerungskomponente und dienen somit auch zur Einstellung eines niedrigeren und milderen pH-Wertes von Waschmitteln. Insbesondere sind hierbei Citronensäure, Bernsteinsäure, Glutarsäure, Adipinsäure, Gluconsäure und beliebige Mischungen aus diesen zu nennen. Mit besonderem Vorzug wird als Gerüstsubstanz die Citronensäure oder Salze der Citronensäure eingesetzt. Weitere besonders bevorzugte Gerüstsubstanzen sind ausgewählt unter Methylglycindiessidsäure (MGDA), Glutaminsäurediacetat (GLDA), Asparaginsäurediacetat (ASDA), Hydroxyethyliminodiacetat (HEIDA), Iminodisuccinat (IDS) und Ethylendiamindisuccinat (EDDS), Carboxymethylinulin und Polyaspartat.

In bevorzugten Ausführungsformen wird als wasserlöslicher, organischer Builder Citronensäure und/oder Citrat eingesetzt. Besonders bevorzugt ist der Einsatz von 0,5 bis 25 Gew.-%, vorzugsweise 0,75 bis 12,5 Gew.-%, weiter bevorzugt 1 bis 4 Gew.-% Citronensäure und/oder 0,5 bis 25 Gew.-%, vorzugsweise 0,75 bis 12,5 Gew.-%, weiter bevorzugt 1 bis 4 Gew.-% Citrat, vorzugsweise Alkalicitrat, noch bevorzugter Natriumcitrat. Citronensäure/Citrat können jeweils in Form ihrer Hydrate eingesetzt werden, so kann z.B. Citronensäure in Form des Monohydrats, Citrat in Form des Trinatriumcitratdihydrats eingesetzt werden.

Als Gerüststoffe sind weiter polymere Polycarboxylate geeignet, dies sind z.B. die Alkalimetallsalze der Polyacrylsäure oder der Polymethacrylsäure, z.B. solche mit einer relativen Molekülmasse von 500 bis 70.000 g/mol. Bei den für polymere Polycarboxylate angegebenen Molmassen handelt es sich im Sinne dieser Anmeldung um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Anmeldung angegebenen Molmassen. Geeignete Polymere sind insbesondere Polyacrylate, die bevorzugt eine Molekülmasse von 2.000 bis 20.000 g/mol aufweisen. Aufgrund ihrer überlegenen Löslichkeit können aus dieser Gruppe wiederum die kurzkettigen Polyacrylate, die Molmassen von 2.000 bis 10.000 g/mol, und besonders bevorzugt von 3.000 bis 5.000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im Allgemeinen 2.000 bis 70.000 g/mol, vorzugsweise 20.000 bis 50.000 g/mol und insbesondere 30.000 bis 40.000 g/mol.

Ein erfindungsgemäßes festes Mittel enthält vorzugsweise mindestens einen wasserlöslichen und/oder wasserunlöslichen, organischen und/oder anorganischen Builder. Zu den wasserlöslichen organischen Buildersubstanzen gehören die oben genannten organischen Gerüstsubstanzen.

Zusätzlich zu den vorstehend genannten wasserlöslichen organischen Buildern, können die Mittel der Erfindung weiterhin auch anorganische wasserlösliche Builder enthalten. Als wasserlösliche anorganische Buildermaterialien kommen insbesondere Alkalisilikate, Alkalicarbonate, Alkalihydrogenbarbonate, Alkaliphosphate und/oder Sesquicarbonate, die in Form ihrer alkalischen, neutralen oder sauren Natrium- oder Kaliumsalze vorliegen können, in Betracht. Ggf. können auch geringe Mengen an Calciumcarbonate in festen Textilwaschmitteln enthalten sein. Geeignet sind z.B. wasserlöslichen kristalline und/oder amorphe Alkalisilikate. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 22, insbesondere 1,9 bis 4 und y eine Zahl von 0 bis 33 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O₅ y H₂O) bevorzugt. Auch aus amorphen Alkalisilikaten hergestellte, praktisch wasserfreie kristalline Alkalisilikate der oben genannten allgemeinen Formel, in der x eine Zahl von 1,9 bis 2,1 bedeutet, können in erfindungsgemäßen Mitteln eingesetzt werden. In einer weiteren Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren Ausführungsform erfindungsgemäßer Mittel eingesetzt. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1. Kristalline schichtförmige Silikate der oben angegebenen Formel (I) werden von der Fa. Clariant GmbH unter dem Handelsnamen Na-SKS vertrieben, z.B. Na-SKS-1 (Na₂Si₂₂O₄₅ x H₂O, Kenyait), Na-SKS-2 (Na₂Si₁₄O₂₉ x H₂O, Magadiit), Na-SKS-3 (Na₂Si₈O₁₇ x H₂O) oder Na-SKS-4 (Na₂Si₄O₉ x H₂O, Makatit). Von diesen eignen sich vor allem Na-SKS-5 (α-Na₂Si₂O₅), Na-SKS-7 (β-Na₂Si₂O₅, Natrosilit), Na-SKS-9 (NaHSi₂O₅ 3 H₂O), Na-SKS-10 (NaHSi₂O₅ 3 H₂O, Kanemit), Na-SKS-11 (t-Na₂Si₂O₅) und Na-SKS-13 (NaHSi₂O₅), insbesondere aber Na-SKS-6 (δ-Na₂Si₂O₅). In einer Ausgestaltung erfindungsgemäßer Mittel setzt man ein granulares Compound aus kristallinem Schichtsilikat und Citrat, aus kristallinem Schichtsilikat und oben genannter (co-)polymerer Polycarbonsäure, oder aus Alkalisilikat und Alkalicarbonat ein, wie es z.B. unter dem Namen Nabion^{®} 15 im Handel erhältlich ist. Derartige wasserlösliche anorganischen Buildermaterialien sind in den erfindungsgemäßen Mitteln vorzugsweise in Mengen von 1 bis 20 Gew.-%, insbesondere 5 bis 15 Gew.-%, enthalten. Des Weiteren sind als wasserlösliche anorganische Buildersubstanzen noch die Carbonate (und hyodrogencarbonate), insbesondere Natriumcarbonat, und die Phosphonsäuren/Phosphonate von Bedeutung. Unter Phosphonsäuren werden dabei auch ggf. substituierte Alkylphosphonsäuren verstanden, die auch mehrere Phosphonsäuregruppierungen aufweisen könne (sogenannte Polyphosphonsäuren). Bevorzugt werden sie ausgewählt aus den Hydroxy- und/oder Aminoalkylphosphonsäuren und/oder deren Alkalisalzen, wie z.B. Dimethylaminomethandiphosphonsäure, 3-Aminopropan-1-hydroxy-1,1-diphosphonsäure, 1-Amino-1-phenylmethandiphosphonsäure, 1-Hydroxyethan-1,1-diphosphonsäure (HEDP), Aminotris-(methylenphosphonsäure), N,N,N',N'-Ethylendiamintetrakis-(methylenphosphonsäure), Diethylentriaminpenta-(methylenphosphonsäure) (DTPMP) und acylierte Derivate der phosphorigen Säure, die auch in beliebigen Mischungen eingesetzt werden können.

In verschiedenen Ausführungsformen setzt sich das Buildersystem vorzugsweise zusammen aus den Komponenten, jeweils bezogen auf die Gesamtmasse des Mittels:
a) 0 bis 10 Gew.-%, vorzugsweise 1 bis 4 Gew.-% Citronensäure; und/oder
b) 0 bis 10 Gew.-%, vorzugsweise 1 bis 4 Gew.-% Citrat, vorzugsweise Alkalicitrat; und/oder
c) 0 bis 40 Gew.-%, vorzugsweise 0 bis 15 Gew.-%, besonders bevorzugt 1 bis 3 Gew.-% Alkalicarbonat, welches auch zumindest anteilig durch Alkalihydrogencarbonat ersetzt sein kann, insbesondere Natriumcarbonat; und/oder
d) 0 bis 20 Gew.-%, vorzugsweise 3 bis 10 Gew.-% Alkalisilikat;
e) 0 bis 10 Gew.-%, vorzugsweise 0,02 bis 2 Gew.-%, besonders bevorzugt 0,5 bis 2 Gew.-% Phosphonsäure und/oder Alkaliphosphonat, insbesondere HEDP und/oder DTPMP; und/oder
f) 0 bis 10 Gew.-%, vorzugsweise 0,5 bis 3 Gew.-% polymeres Polycarboxylat, insbesondere Polyacrylat.

Hinsichtlich der Komponente f) sind z.B. die Alkalimetallsalze der Polyacrylsäure oder der Polymethacrylsäure, z.B. solche mit einer relativen Molekülmasse von 500 bis 70.000 g/mol geeignet. Diese Substanzklasse wurde im Detail bereits weiter oben beschrieben. Die (co-)polymeren Polycarboxylate können entweder als Pulver oder als wässrige Lösung eingesetzt werden.

Die erfindungsgemäßen Mittel sind vorzugsweise frei von Phosphat-Builder, d.h. enthalten weniger als 1 Gew.-% bevorzugt keinen bewusst zugegebenen Phosphat-Builder.

Die Mittel können ferner auch wasserunlösliche Buildersubstanzen enthalten. Als wasserunlösliche anorganische Buildermaterialien werden insbesondere kristalline oder amorphe wasserdispergierbare Alkalialumosilikate, in Mengen von bis zu 50 Gew.-%, vorzugsweise nicht über 40 Gew.-%, insbesondere von 3 bis 20 Gew.-% und besonders bevorzugt von 1 bis 15 Gew.- %, eingesetzt. Unter diesen sind die kristallinen Natriumalumosilikate in Waschmittelqualität, insbesondere Zeolith A, Zeolith P, Zeolith MAP und ggf. Zeolith X, allein oder in Mischungen, z.B. in Form eines Co-Kristallisats aus den Zeolithen A und X (Vegobond^{®} AX, ein Handelsprodukt der Condea Augusta S.p.A.), bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das gemäß DE 2412837 A1 bestimmt werden kann, liegt in der Regel im Bereich von 100 bis 200 mg CaO pro Gramm.

In Ergänzung zu den zuvor beschriebenen Gerüststoffen können in dem Waschmittel reinigungsaktive Polymere enthalten sein. Der Gewichtsanteil der reinigungsaktiven Polymere am Gesamtgewicht erfindungsgemäßer Waschmittel beträgt vorzugsweise 0,1 bis 20 Gew.-%, vorzugsweise 1,0 bis 15 Gew.-% und weiter bevorzugt 2,0 bis 12 Gew.-%. Als reinigungsaktive Polymere werden vorzugsweise Sulfonsäuregruppen-haltige Polymere, insbesondere aus der Gruppe der copolymeren Polysulfonate, eingesetzt. Diese copolymeren Polysulfonate enthalten neben Sulfonsäuregruppen-haltigem(n) Monomer(en) wenigstens ein Monomer aus der Gruppe der ungesättigten Carbonsäuren. Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist. Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren. Bei den Sulfonsäuregruppen-haltigen Monomeren sind solche der Formel R⁵(R⁶)C=C(R¹)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für -COOH oder -COOR⁴ steht, wobei R⁴ ein gesättigter oder ungesättigter, geradkettiger oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₂CH₃)-. Unter diesen Monomeren bevorzugt sind solche der Formeln H₂C=CH-X-SO₃H, H₂C=C(CH₃)-X-SO₃H und HO₃S-X-(R⁶)C=C(R⁷)-X-SO₃H, in denen R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, - COO-(CH₂)ₖ- mit k = 1 bis 6, -C(O)-NH-C(CH₃)₂-,-C(O)-NH-C(CH₃)₂-CH₂- und -C(O)-NH-CH(CH₂CH₃)-. Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)-propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze. In den Polymeren können die Sulfonsäuregruppen ganz oder teilweise in neutralisierter Form vorliegen. Der Einsatz von teil- oder vollneutralisierten sulfonsäuregruppenhaltigen Copolymeren ist erfindungsgemäß bevorzugt. Die Molmasse der erfindungsgemäß bevorzugt eingesetzten Sulfo-Copolymere kann variiert werden, um die Eigenschaften der Polymere dem gewünschten Verwendungszweck anzupassen. Als nichtionische Monomere werden vorzugsweise Monomere der Formel R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht. Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie z.B. 1-Decen, 1-Dodecen, 1-Hexadecen, 1-Oktadecen und C₂₂-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2-Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)-acrylamid, Acrylsäure-2-Ethylhexylester, Methacrylsäure-2-Ethylhexylester, N-(2-Ethylhexyl)-acrylamid, Acrylsäureoctylester, Methacrylsäureoctylester, N-(Octyl)-acrylamid, Acrylsäurelaurylester, Methacrylsäurelaurylester, N-(Lauryl)-acrylamid, Acrylsäurestearylester, Methacrylsäurestearylester, N-(Stearyl)-acrylamid, Acrylsäurebehenylester, Methacrylsäurebehenylester und N-(Behenyl)-acrylamid oder deren Mischungen. Der Gewichtsanteil der Sulfonsäuregruppen-haltigen Copolymere am Gesamtgewicht erfindungsgemäßer Waschmittel beträgt vorzugsweise 0,1 bis 15 Gew.-%, vorzugsweise 1,0 bis 12 Gew.-% und weiter bevorzugt 2,0 bis 10 Gew.-%.

In einer Ausführungsform umfasst ein Waschmittel,
- 5 bis 70 Gew.-%, insbesondere 5 bis 30 Gew.-% Tenside und/oder
- 0 bis 10 Gew.-%, insbesondere 1 bis 3 Gew.-% wasserlösliches oder wasserdispergierbares anorganisches Buildermaterial und/oder
- 0 bis 10 Gew.-%, insbesondere 1 bis 4 Gew.-%, wasserlösliche organische Buildersubstanzen und/oder
- 0,01 bis 5 Gew.-% Komplexbildner und/oder
- 0,01 bis 5 Gew.-% Vergrauungsinhibitor und/oder
- 0,01 bis 5 Gew.-% Schauminhibitor, und/oder
- 0,01 bis 5 Gew.-% optische Aufheller.

In einer bevorzugten Ausführungsform umfasst ein flüssiges Waschmittel,
- 5 bis 20 Gew.-%, insbesondere 9 bis 17 Gew.-% anionische Tenside und/oder
- 1 bis 20 Gew.-%, insbesondere 5 bis 9 Gew.-% nichtionische Tenside und/oder
- 0,5 bis 10 Gew.-%, insbesondere 1 bis 4 Gew.-%, wasserlösliche organische Buildersubstanzen, insbesondere Citronensäure oder Citrat, und/oder
- 0,01 bis 5 Gew.-%, insbesondere 0,5 bis 2 Gew.-% Komplexbildner, insbesondere HEDP, und/oder
- 0,01 bis 5 Gew.-%, insbesondere 0,2 bis 1 Gew.-% Vergrauungsinhibitor und/oder
- 0,01 bis 5 Gew.-% Schauminhibitor.

In einer weiteren bevorzugten Ausführungsform umfasst ein flüssiges Waschmittel,
- 0 bis 10 Gew.-%, insbesondere 1 bis 3 Gew.-% anionische Tenside und/oder
- 5 bis 20 Gew.-%, insbesondere 10 bis 18 Gew.-% nichtionische Tenside und/oder
- 1 bis 10 Gew.-%, insbesondere 1,5 bis 3 Gew.-% wasserlösliches oder wasserdispergierbares anorganisches Buildermaterial und/oder
- 0 bis 1 Gew.-%, insbesondere 0,01 bis 0,5 Gew.-% Duftstoffe und/oder
- 0,01 bis 5 Gew.-%, insbesondere 0,05 bis 0,5 Gew.-% optische Aufheller.

Geeignete Vergrauungsinhibitoren bzw. soil-release-Wirkstoffe (soil release polymer) sind Celluloseether, wie Carboxymethylcellulose, Methylcellulose, Hydroxyalkylcellulosen und Cellulosemischether, wie Methylhydroxyethylcellulose, Methylhydroxypropylcellulose und Methylcarboxymethylcellulose. Vorzugsweise werden Natriumcarboxymethylcellulose, Hydroxyporpylmethylcellulose und deren Gemische und ggf. deren Gemische mit Methylcellulose eingesetzt. Zu den üblicherweise eingesetzten Soil-release-Wirkstoffen gehören Copolyester, die Dicarbonsäureeinheiten, Alkylenglykoleinheiten und Polyalkylenglykoleinheiten enthalten. Der Anteil an Vergrauungsinhibitoren und/oder soil-release-Wirkstoffen in erfindungsgemäßen Mitteln liegt im Allgemeinen nicht über 2 Gew.-% und beträgt vorzugsweise 0,5 bis 1,5 Gew.-%, besonders bevorzugt 0,5 bis 2 Gew.-%.

Als optische Aufheller für insbesondere Textilien aus Cellulosefasern (z.B. Baumwolle) können z.B. Derivate der Diaminostilbendisulfonsäure bzw. deren Alkalimetallsalze enthalten sein. Geeignet sind z.B. Salze der 4,4'-Bis(2-anilino-4-morpholino-1,3,5-triazin-6-yl-amino)-stilben-2,2'-disulfonsäure oder gleichartig aufgebaute Verbindungen, die anstelle der Morpholinogruppe eine Diethanolaminogruppe, eine Methylaminogruppe oder eine 2-Methoxyethylaminogruppe tragen. Weiterhin können Aufheller vom Typ des substituierten 4,4'-Distyryl-diphenyl anwesend sein, z.B. 4,4'-Bis-(4-chlor-3-sulfostyryl)-diphenyl. Auch Gemische von Aufhellern können verwendet werden. Für Polyamidfasern eignen sich besonders gut Aufheller vom Typ der 1,3-Diaryl-2-pyrazoline, z.B. 1-(p-Sulfoamoylphenyl)-3-(p-chlorphenyl)-2-pyrazolin sowie gleichartig aufgebaute Verbindungen. Der Gehalt des Mittels an optischen Aufhellern bzw. Aufhellergemischen liegt im Allgemeinen nicht über 1 Gew.-%, vorzugsweise 0,05 bis 0,5 Gew.-%. In einer bevorzugten Ausführungsform der Erfindung ist das Mittel frei von derartigen Wirkstoffen.

Zu den in den erfindungsgemäßen Mitteln einsetzbaren üblichen Schaumregulatoren gehören z.B. Polysiloxan-Kieselsäure-Gemische, wobei die darin enthaltene feinteilige Kieselsäure vorzugsweise silaniert oder anderweitig hydrophobiert ist. Die Polysiloxane können sowohl aus linearen Verbindungen wie auch aus vernetzten Polysiloxan-Harzen sowie aus deren Gemischen bestehen. Weitere Entschäumer sind Paraffinkohlenwasserstoffe, insbesondere Mikroparaffine und Paraffinwachse, deren Schmelzpunkt oberhalb 40°C liegt, gesättigte Fettsäuren bzw. Seifen mit insbesondere 20 bis 22 C-Atomen, z.B. Natriumbehenat, und Alkalisalze von Phosphorsäuremono- und/oder -dialkylestern, in denen die Alkylketten jeweils 12 bis 22 C-Atome aufweisen. Unter diesen wird bevorzugt Natriummonoalkylphosphat und/oder -dialkylphosphat mit C₁₆₋₁₈-Alkylgruppen eingesetzt. Der Anteil der Schaumregulatoren kann vorzugsweise 0,2 bis 2 Gew.-%, besonders bevorzugt nicht mehr als 1 Gew.-% betragen.

Zur Einstellung des gewünschten pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, vorzugsweise Natriumhydroxid, enthalten. Derartige pH-Regulatoren sind in den erfindungsgemäßen Mitteln vorzugsweise nicht über 10 Gew.-%, insbesondere von 0,5 bis 6 Gew.-%, besonders bevorzugt von 0,3 bis 2 Gew.-% enthalten.

Als für den Einsatz in erfindungsgemäßen Mitteln geeignete Persauerstoffverbindungen kommen insbesondere organische Persäuren bzw. persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, zu denen Perborat, Percarbonat, Persilikat und/oder Persulfat wie Caroat gehören, sowie Wasserstoffperoxid-Einschlussverbindungen, wie H₂O₂-Harnstoffaddukte, in Betracht. Wasserstoffperoxid kann dabei auch mit Hilfe eines enzymatischen Systems, d.h. einer Oxidase und ihres Substrates, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Die Persauerstoffverbindungen können als solche oder in Form diese enthaltender Mittel, die prinzipiell alle üblichen Wasch-, Reinigungs- oder Desinfektionsmittelbestandteile enthalten können, zu der Waschlauge zugegeben werden. Besonders bevorzugt wird Alkalipercarbonat oder Alkaliperborat-Monohydrat eingesetzt. Falls ein erfindungsgemäßes Mittel Persauerstoffverbindungen enthält, sind diese in Mengen von vorzugsweise bis zu 50 Gew.-%, insbesondere von 5 bis 30 Gew.-%, weiter bevorzugt von 0,1 bis 20 Gew.-% vorhanden.

Als Bleichaktivatoren können in den Mitteln Verbindungen, die unter Perhydrolysebedingungen aliphatische Peroxocarbonsäuren mit vorzugsweise 1 bis 10 C-Atomen, insbesondere 2 bis 4 C-Atomen, und/oder ggf. substituierte Perbenzoesäure ergeben, eingesetzt werden. Geeignet sind Substanzen, die O- und/oder N-Acylgruppen der genannten C-Atomzahl und/oder ggf. substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate oder -carboxylate bzw. die Sulfon- oder Carbonsäuren von diesen, insbesondere Nonanoyl- oder Isononanoyloxybenzolsulfonat oder Laroyloxybenzolsulfonat (NOBS bzw. iso-NOBS bzw. LOBS), 4-(2-Decanoyloxyethoxycarbonyloxy)-benzolsulfonat (DECOBS) oder Decanoyloxybenzoat (DOBA), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol bzw. deren beschriebene Mischungen (SORMAN), acylierte Zuckerderivate, insbesondere Pentaacetylglukose (PAG), Pentaacetylfruktose, Tetraacetylxylose und Octaacetyllactose, acetyliertes, ggf. N-alkyliertes Glucamin und Gluconolacton, N-acylierte Lactame, z.B. N-Benzoylcaprolactam, Nitrile, aus denen sich Perimidsäuren bilden, insbesondere Aminoacetonitrilderivate mit quaterniertem Stickstoffatom, und/oder sauerstoffübertragende Sulfonimine und/oder Acylhydrazone. Die hydrophil substituierten Acylacetale und die Acyllactame werden ebenfalls bevorzugt eingesetzt. Auch Kombinationen konventioneller Bleichaktivatoren können eingesetzt werden. Derartige Bleichaktivatoren können, insbesondere bei Anwesenheit obengenannter Wasserstoffperoxid-liefernder Bleichmittel, im üblichen Mengenbereich, vorzugsweise in Mengen von 0,5 bis 10 Gew.-%, insbesondere 1 bis 8 Gew.-%, bezogen auf gesamtes Mittel, enthalten sein, fehlen bei Einsatz von Percarbonsäure als alleinigem Bleichmittel jedoch vorzugsweise ganz.

Zusätzlich zu den konventionellen Bleichaktivatoren oder an deren Stelle können in festen Mitteln auch Sulfonimine und/oder bleichverstärkende Übergangsmetallsalze bzw. Übergangsmetallkomplexe als sogenannte Bleichkatalysatoren enthalten sein.

Als einen weiteren Bestandteil können die erfindungsgemäßen Waschmittel ein organisches Lösungsmittel enthalten. Der Zusatz organischer Lösungsmittel wirkt sich vorteilhaft auf die Enzymstabilität und die Reinigungsleistung dieser Mittel aus. Bevorzugte organische Lösungsmittel stammen aus der Gruppe ein- oder mehrwertigen Alkohole, Alkanolamine oder Glykolether. Vorzugsweise werden die Lösungsmittel ausgewählt aus Ethanol, n- oder i-Propanol, Butanol, Glykol, Propandiol, Butandiol, Glycerin, Diglykol, Propyldiglykol, Butyldiglykol, Hexylenglycol, Ethylenglykolmethylether, Ethylenglykolethylether, Ethylenglykolpropylether, Etheylenglykolmonon-butylether, Diethylenglykolmethylether, Di-ethylenglykolethylether, Propylenglykolmethylether, Propylenglykolethylether, Propylenglykolpropylether, Dipropylenglykolmethylether, Dipropylenglykolethylether, Methoxytriglykol, Ethoxytriglykol, Butoxytriglykol, 1-Butoxyethoxy-2-propanol, 3-Methyl-3-methoxybutanol, Propylenglykol-t-butylether sowie Mischungen dieser Lösungsmittel. Der Gewichtsanteil dieser organischen Lösungsmittel am Gesamtgewicht erfindungsgemäßer Waschmittel beträgt vorzugsweise 0,1 bis 10 Gew.-%, bevorzugt 0,2 bis 8,0 Gew.-% und weiter bevorzugt 0,5 bis 5,0 Gew.-%. Ein besonders bevorzugtes und in Bezug auf die Stabilisierung der Waschmittel besonders wirksames organisches Lösungsmittel ist das Glycerin sowie das 1,2 Propylenglykol. Flüssige Waschmittel umfassen vorzugsweise mindestens ein Polyol, vorzugsweise aus der Gruppe Glycerin und 1,2-Propylenglycol, bezogen auf das Gesamtgewicht des Waschmittels, vorzugsweise zu 0,1 bis 10 Gew.-%, bevorzugt 0,2 bis 8,0 Gew.-% und weiter bevorzugt 0,5 bis 5,0 Gew.-%. Weitere bevorzugte organische Lösungsmittel sind die organischen Amine und Alkanolamine. Die erfindungsgemäßen Waschmittel enthalten diese Amine vorzugsweise in Mengen von 0,1 bis 10 Gew.-%, bevorzugt von 0,2 bis 8,0 Gew.-% und weiter bevorzugt von 0,5 bis 5,0 Gew.-%, jeweils bezogen auf ihr Gesamtgewicht. Ein besonders bevorzugtes Alkanolamin ist das Ethanolamin.

Ein weiterer bevorzugter Bestandteil der erfindungsgemäßen Waschmittel ist ein Zuckeralkohol (Alditol). Die Gruppe der Alditole umfasst nichtcyclische Polyole der Formel HOCH₂[CH(OH)]ₙCH₂OH. Zu den Alditolen zählen z.B. Mannit (Mannitol), Isomalt, Lactit, Sorbit (Sorbitol) und Xylit (Xylitol), Threit, Erythrit und Arabit. Als in Bezug auf die Enzymstabilität besonders vorteilhaft hat sich das Sorbitol erwiesen. Der Gewichtsanteil des Zuckeralkohols am Gesamtgewicht des Waschmittels beträgt vorzugsweise 1,0 bis 10 Gew.-%, bevorzugt 2,0 bis 8,0 Gew.-% und weiter bevorzugt 3,0 bis 6,0 Gew.-%.

Erfindungsgemäße Waschmittel können ausschließlich eine Protease, wie hierin definiert, enthalten. Alternativ können sie auch weitere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder andere - von den erfindungsgemäßen Protease unterscheidbare - Protease, sowie deren Gemische. Weitere Enzyme sind in dem Mittel vorteilhafterweise jeweils in einer Menge von 1 x 10⁻⁸ bis 5 Gew.-% bezogen auf aktives Protein enthalten. Zunehmend bevorzugt ist jedes weitere Enzym in einer Menge von 1 x 10⁻⁷ bis 3 Gew.- %, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels, darunter insbesondere zwischen der genannten Protease und einer Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten. Erfindungsgemäß bevorzugte Textilwaschmittel weisen mindestens eine Protease und mindestens eine Amylase auf. In einer weiteren bevorzugten Ausführungsform der Erfindung weisen Textilwaschmittel mindestens eine Protease und mindestens eine Cellulase auf. In einer weiteren bevorzugten Ausführungsform weisen Textilwaschmittel mindestens eine Protease und mindestens eine Lipase auf. Besonders bevorzugt sind Textilwaschmittel, welche 3 bis 10 verschiedene Enzyme aufweisen, wobei Textilwaschmittel, welche 3 bis 10 unterschiedliche Enzymarten aufweisen, im Hinblick auf die Reinigungsleistung gegenüber einem sehr großen Fleckenspektrum von besonderer Bevorzugung sein können.

Beispiele für Proteasen sind die Subtilisine BPN' aus *Bacillus amyloliquefaciens* und Carlsberg aus *Bacillus licheniformis,* die Protease PB92, die Subtilisine 147 und 309, die Protease aus *Bacillus lentus,* Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von dem Unternehmen Novozymes erhältlich. Die Subtilisine 147 und 309 werden unter den Handelsnamen Esperase^{®} bzw. Savinase^{®} von dem Unternehmen Novozymes vertrieben. Von der Protease aus *Bacillus lentus* DSM 5483 leiten sich Proteasevarianten ab. Weitere brauchbare Proteasen sind z.B. die unter den Handelsnamen Durazym^{®}, Relase^{®}, Everlase^{®}, Nafizym^{®}, Natalase^{®}, Kannase^{®}, Progress Uno 101L^{®} und Ovozyme^{®} von dem Unternehmen Novozymes, die unter den Handelsnamen, Purafect^{®}, Purafect^{®} OxP, Purafect^{®} Prime, Excellase^{®}, Properase^{®}, Preferenz P100^{®} und Preferenz P300^{®} von dem Unternehmen Danisco/DuPont, das unter dem Handelsnamen Lavergy pro 104 LS^{®} von dem Unternehmen BASF, das unter dem Handelsnamen Protosol^{®} von dem Unternehmen Advanced Biochemicals Ltd., das unter dem Handelsnamen Wuxi^{®} von dem Unternehmen Wuxi Snyder Bioproducts Ltd., die unter den Handelsnamen Proleather^{®} und Protease P^{®} von dem Unternehmen Amano Pharmaceuticals Ltd., und das unter der Bezeichnung Proteinase K-16 von dem Unternehmen Kao Corp. erhältlichen Enzyme. Besonders bevorzugt eingesetzt werden auch die Proteasen aus *Bacillus gibsonii* und *Bacillus pumilus,* die offenbart sind in den internationalen Patentanmeldungen WO 2008/086916 A1 und WO 2007/131656 A1. Weitere vorteilhaft einsetzbare Proteasen sind offenbart in den Patentanmeldungen WO 91/02792 A1, WO 2008/007319 A2, WO 93/18140 A1, WO 01/44452 A1, GB 1243784 A, WO 96/34946 A1, WO 02/029024 A1 und WO 03/057246 A1. Weitere verwendbare Proteasen sind diejenigen, die in den Mikroorganismen *Stenotrophomonas maltophilia,* insbesondere *Stenotrophomonas maltophilia* K279a, *Bacillus intermedius* sowie *Bacillus sphaericus* natürlicherweise vorhanden sind.

Beispiele für Amylasen sind die α-Amylasen aus *Bacillus licheniformis, Bacillus amyloliquefaciens* oder *Bacillus stearothermophilus* sowie insbesondere auch deren für den Einsatz in Wasch- oder Reinigungsmitteln verbesserte Weiterentwicklungen. Das Enzym aus *Bacillus licheniformis* ist von dem Unternehmen Novozymes unter dem Namen Termamyl^{®} und von dem Unternehmen Danisco/DuPont unter dem Namen Purastar^{®} ST erhältlich. Weiterentwicklungsprodukte dieser α-Amylase sind unter den Handelsnamen Duramyl^{®} und Termamyl^{®} ultra (beide von Novozymes), Purastar^{®} OxAm (Danisco/DuPont) und Keistase^{®} (Daiwa Seiko Inc.) erhältlich. Die α-Amylase von *Bacillus amyloliquefaciens* wird von dem Unternehmen Novozymes unter dem Namen BAN^{®} vertrieben, und abgeleitete Varianten von der α-Amylase aus *Bacillus stearothermophilus* unter den Namen BSG^{®} und Novamyl^{®}, ebenfalls von dem Unternehmen Novozymes. Des Weiteren sind für diesen Zweck die α-Amylase aus *Bacillus sp.* A 7-7 (DSM 12368) und die Cyclodextrin-Glucanotransferase (CGTase) aus *Bacillus agaradherens* (DSM 9948) hervorzuheben. Ferner sind die amylolytischen Enzyme einsetzbar, die in den internationalen Patentanmeldungen WO 95/26397 A1, WO 96/23873 A1, WO 99/23211 A1, WO 00/60060 A2, WO 03/002711 A2, WO 03/054177 A2, WO 2006/002643 A2, WO 2007/079938 A2, WO 2011/100410 A2 und WO 2013/003659 A1 offenbart sind. Ebenso sind Fusionsprodukte aller genannten Moleküle einsetzbar. Darüber hinaus sind die unter den Handelsnamen Fungamyl^{®} von dem Unternehmen Novozymes erhältlichen Weiterentwicklungen der α-Amylase aus *Aspergillus niger* und A. *oryzae* geeignet. Weitere vorteilhaft einsetzbare Handelsprodukte sind z.B. die Amylase-LT^{®} und Stainzyme^{®} oder Stainzyme^{®} ultra bzw. Stainzyme^{®} plus sowie Amplify^{™} 12L oder Amplify Prime^{™} 100L, letztere ebenfalls von dem Unternehmen Novozymes, sowie die PREFERENZ S^{®} Serie von dem Unternehmen Danisco/DuPont, umfassend z.B. PREFERENZ S100^{®}, PREFERENZ S1000^{®} oder PREFERENZ S210^{®}. Auch durch Punktmutationen erhältliche Varianten dieser Enzyme können erfindungsgemäß eingesetzt werden.

Bevorzugte Amylasen umfassen a) eine α-Amylase, die eine Aminosäuresequenz umfasst, die zu der in SEQ ID NO:2 angegebenen Aminosäuresequenz über deren Gesamtlänge zu mindestens 80%, zunehmend bevorzugt zu mindestens 81%, 82, 83%, 84%, 85%, 86%, 87%, 88%, 89%, 90%, 91%, 92%, 93%, 94%, 95%, 96%, 97%, 98%, 99%, identisch ist und mindestens eine Aminosäuresubstitution an einer der Positionen 172, 202, 208, 255 und 261 in der Zählung gemäß SEQ ID NO:2 aufweist, vorzugsweise ausgewählt aus der aus M202L, M202V, M202S, M202T, M202I, M202Q, M202W, S255N, R172Q und Kombinationen davon bestehenden Gruppe. Besonders bevorzugt umfasst die Amylase die Aminosäuresubstitution M202L oder M202T.

Weiter bevorzugte Amylasen umfassen b) eine α-Amylase, die eine Aminosäuresequenz umfasst, die zu der in SEQ ID NO:3 angegebenen Aminosäuresequenz über deren Gesamtlänge zu mindestens 60%, zunehmend bevorzugt zu mindestens 70%, 75%, 80%, 81%, 82%, 83%, 84%, 85%, 86%, 87%, 88%, 89%, 90%, 91%, 92%, 93%, 94%, 95%, 96%, 97%, 98%, 99%, identisch ist und mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:3 aufweist. Besonders bevorzugte Amylasen weisen mindestens eine, Aminosäuresubstitution an einer der Positionen 9, 26, 149, 182, 186, 202, 257, 295, 299, 323, 339 and 345, und/oder besonders bevorzugt mindestens eine Aminosäuresubstitution oder -deletion, die aus der aus R118K, D183*, G184*, N195F, R320K, R458K und Kombinationen davon bestehenden Gruppe ausgewählt ist. Ganz besonders bevorzugte Amylasen umfassen eine Aminosäuresequenz, die zu der in SEQ ID NO:3 angegebenen Aminosäuresequenz über deren Gesamtlänge zu mindestens 60%, zunehmend bevorzugt zu mindestens 70%, 75%, 80%, 81%, 82%, 83%, 84%, 85%, 86%, 87%, 88%, 89%, 90%, 91%, 92%, 93%, 94%, 95%, 96%, 97%, 98%, 99%, identisch ist, und weisen eines der folgenden Aminosäuresubstitutions/-deletionskombinationen auf:
(i) M9L+M323T;
(ii) M9L+M202L/T/V/I+M323T;
(iii) M9L+N195F+M202L/T/V/I+M323T;
(iv) M9L+R118K+D183*+G184*+R320K+M323T+R458K;
(v) M9L+R118K+D183*+G184*+M202L/T/V/I+R320K+M323T+R458K;
(vi) M9L+G149A+G182T+G186A+M202L+T2571+Y295F+N299Y+M323T+A339S+E345R;
(vii) M9L+G149A+G182T+G186A+M2021+T257I+Y295F+N299Y+M323T+A339S+E345R;
(viii) M9L+R118K+G149A+G182T+D183*+G184*+G1186A+M202L+T257I+Y295F+N299Y+R320K +M323T+A339S+E345R+R458K;
(ix) M9L+R118K+G149A+G182T+D183*+G184*+G1186A+M202I+T257I+Y295F+N299Y+R320K +M323T+A339S+E345R+R458K;
(x) M9L+R118K+D183*+D184*+N195F+M202L+R320K+M323T+R458K;
(xi) M9L+R118K+D183*+D184*+N195F+M202T+R320K+M323T+R458K;
(xii) M9L+R118K+D183*+D184*+N195F+M202I+R320K+M323T+R458K;
(xiii) M9L+R118K+D183*+D184*+N195F+M202V+R320K+M323T+R458K;
(xiv) M9L+R118K+N150H+D183*+D184*+N195F+M202L+V214T+R320K+M323T+R458K;
(xv) M9L+R118K+D183*+D184*+N195F+M202L+V214T+R320K+M323T+E345N+R458K.

Weiter bevorzugte Amylasen umfassen c) eine α-Amylase, die eine Aminosäuresequenz umfasst, die zu der in SEQ ID NO:4 angegebenen Aminosäuresequenz über deren Gesamtlänge zu mindestens 90% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:4, aufweist, vorzugsweise Aminosäuredeletionen an den Positionen 183 und 184. Bevorzugte Aminosäuresubstitutionen umfassen E260A/D/C/Q/L/M/F/P/S/W/V/G/H/I/K/N/R/T/Y, G304R/K/E/Q, W140Y/F, W189E/G/T, D134E, F262G/P, W284D/H/F/Y/R, W347H/F/Y, W439R/G, G476E/Q/R/K, G477E/Q/K/M/R, N195F/Y, N197F/L, Y198N, Y200F, Y203F, I206H/L/N/F/Y, H210Y, E212V/G, V213A, M116T, Q129L, G133E, E134Y, K142R, P146S, G147E, G149R, N151R, Y152H, Q169E, N174R, A186R, Y243F, S244Q, G303V, R320N, R359I, N418D und A447V.

Beispiele für Cellulasen (Endoglucanasen, EG) ist die pilzliche, Endoglucanase-(EG)-reiche Cellulase-Präparation bzw. deren Weiterentwicklungen, die von dem Unternehmen Novozymes unter dem Handelsnamen Celluzyme^{®} angeboten wird. Die ebenfalls von dem Unternehmen Novozymes erhältlichen Produkte Endolase^{®} und Carezyme^{®} basieren auf der 50 kD-EG, bzw. der 43 kD-EG aus *Humicola insolens* DSM 1800. Weitere einsetzbare Handelsprodukte dieses Unternehmens sind Cellusoft^{®}, Renozyme^{®} und Celluclean^{®}. Weiterhin einsetzbar sind z.B. Cellulasen, die von dem Unternehmen AB Enzymes unter den Handelsnamen Ecostone^{®} und Biotouch^{®} erhältlich sind, und die zumindest zum Teil auf der 20 kD-EG aus *Melanocarpus* basieren. Weitere Cellulasen von dem Unternehmen AB Enzymes sind Econase^{®} und Ecopulp^{®}. Weitere geeignete Cellulasen sind aus *Bacillus sp.* CBS 670.93 und CBS 669.93, wobei die aus *Bacillus sp.* CBS 670.93 von dem Unternehmen Danisco/DuPont unter dem Handelsnamen Puradax^{®} erhältlich ist. Weitere verwendbare Handelsprodukte des Unternehmens Danisco/DuPont sind "Genencor detergent cellulase L" und IndiAge^{®} Neutra.

Als weitere Enzyme einsetzbar sind z.B. Lipasen oder Cutinasen, insbesondere wegen ihrer Triglycerid-spaltenden Aktivitäten, aber auch, um aus geeigneten Vorstufen *in situ* Persäuren zu erzeugen. Hierzu gehören z.B. die ursprünglich aus *Humicola lanuginosa (Thermomyces lanuginosus*) erhältlichen bzw. daraus weiterentwickelten Lipasen, insbesondere solche mit einem oder mehreren der folgenden Aminosäureaustausche ausgehend von der genannten Lipase in den Positionen D96L, T213R und/oder N233R, besonders bevorzugt T213R und N233R. Lipasen werden z.B. von dem Unternehmen Novozymes unter den Handelsnamen Lipolase^{®}, Lipolase^{®} Ultra, LipoPrime^{®}, Lipozyme^{®} und Lipex^{®} vertrieben. Eine weitere vorteilhaft einsetzbare Lipase ist unter dem Handelsnamen Lipoclean^{®} von dem Unternehmen Novozymes erhältlich. Des Weiteren sind z.B. die Cutinasen einsetzbar, die ursprünglich aus *Fusarium solani pisi* und *Humicola insolens* isoliert worden sind. Ebenso brauchbare Lipasen sind von dem Unternehmen Amano unter den Bezeichnungen Lipase CE^{®}, Lipase P^{®}, Lipase B^{®} bzw. Lipase CES^{®}, Lipase AKG^{®}, *Bacillus sp.* Lipase^{®}, Lipase AP^{®}, Lipase M-AP^{®} und Lipase AML^{®} erhältlich. Von dem Unternehmen Danisco/DuPont sind z.B. die Lipasen bzw. Cutinasen einsetzbar, deren Ausgangsenzyme ursprünglich aus *Pseudomonas mendocina* und *Fusarium solanii* isoliert worden sind. Als weitere wichtige Handelsprodukte sind die von dem Unternehmen Danisco/DuPont vertriebenen Präparationen M1 Lipase^{®} und Lipomax^{®} und die von dem Unternehmen Meito Sangyo KK unter den Namen Lipase MY-30^{®}, Lipase OF^{®} und Lipase PL^{®} vertriebenen Enzyme zu erwähnen, ferner das Produkt Lumafast^{®} von dem Unternehmen Danisco/DuPont.

Zur Erhöhung der bleichenden Wirkung können erfindungsgemäß Oxidoreduktasen, z.B. Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Die im Rahmen der vorliegenden Erfindung einzusetzenden Enzyme können z.B. ursprünglich aus Mikroorganismen, etwa der Gattungen *Bacillus, Streptomyces, Humicola* oder *Pseudomonas,* stammen und/oder nach an sich bekannten biotechnologischen Verfahren durch geeignete Mikroorganismen produziert werden, etwa durch transgene Expressionswirte, z.B. der Gattungen *Escherichia, Bacillus* oder durch filamentöse Pilze. Es wird betont, dass es sich insbesondere auch um technische Enzympräparationen des jeweiligen Enzyms handeln kann, d.h. Begleitstoffe vorliegen können. Daher können die Enzyme zusammen mit Begleitstoffen, etwa aus der Fermentation oder mit weiteren Stabilisatoren, konfektioniert und eingesetzt werden.

Ein erfindungsgemäßes Mittel enthält die Protease vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro Gramm des Mittels. In verschiedenen Ausführungsformen beträgt die Konzentration der hierin beschriebenen Protease (aktives Enzym) in dem Mittel >0 bis 1 Gew.-%, vorzugsweise 0,001 bis 0,1 Gew.-% bezogen auf das Gesamtgewicht des Mittels.

Ferner kann die in dem Mittel enthaltene Protease und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Waschmittel selbst in einem Behältnis, vorzugsweise einem luftdurchlässigen Behältnis, verpackt sein, aus dem es kurz vor Gebrauch oder während des Waschvorgangs freigesetzt wird.

Die Protease und/oder ggf. weitere in dem Waschmittel enthaltene Enzyme gleicher oder anderer Art kann/können an Trägerstoffen adsorbiert und/oder in Hüllsubstanzen eingebettet sein, um sie gegen vorzeitige Inaktivierung zu schützen. In der Waschflotte, also unter Anwendungsbedingungen, wird das Enzym dann freigesetzt und kann seine katalytische Wirkung entfalten. Bevorzugte Konfektionierungsformen weisen 0,05 bis 15 Gew.-% und insbesondere bis 10 Gew.-% aktives Protein der genannten Protease auf.

Ein erfindungsgemäßes Waschmittel enthält die Protease zunehmend bevorzugt in einer Menge von 1 x 10⁻⁸ bis 5 Gew.-%, von 0,0001 bis 1 Gew.-%, von 0,0005 bis 0,5 Gew.-%, von 0,001 bis 0,1 Gew.-%, jeweils bezogen auf aktives Protein.

In den hierin beschriebenen Mitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt. Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen z.B. die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt. Alternativ können die Enzyme sowohl für die feste als auch für die flüssige Darreichungsform verkapselt werden, z.B. durch Sprühtrocknung oder Extrusion der Enzymlösung zusammen mit einem vorzugsweise natürlichen Polymer oder in Form von Kapseln, z.B. solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, z.B. Stabilisatoren, Emulgatoren, Pigmente, Bleich- oder Farbstoffe aufgebracht werden. Derartige Kapseln werden nach an sich bekannten Methoden, z.B. durch Schüttel- oder Rollgranulation oder in Fluid-bed-Prozessen aufgebracht. Vorteilhafterweise sind derartige Granulate, z.B. durch Aufbringen polymerer Filmbildner, staubarm und aufgrund der Beschichtung lagerstabil. Weiterhin ist es möglich, zwei oder mehrere Enzyme zusammen zu konfektionieren, so dass ein einzelnes Granulat mehrere Enzymaktivitäten aufweist.

Die Enzyme können auch in wasserlösliche Filme, wie sie z.B. bei der Konfektionierung von Waschmitteln in Einheitsdosisform verwendet werden, eingebracht werden. Ein derartiger Film ermöglicht die Freisetzung der Enzyme nach Kontakt mit Wasser. Wie hierin verwendet, bezieht sich "wasserlöslich" auf eine Filmstruktur, die vorzugsweise vollständig wasserlöslich ist. Bevorzugt besteht ein solcher Film aus (vollständig oder teilweise hydrolysiertem) Polyvinylalkohol (PVA).

Die Ausführungsformen der vorliegenden Erfindung umfassen alle festen, pulverförmigen, granularen, tablettenförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 bis 1.200 g/l, insbesondere 500 bis 900 g/l oder 600 bis 850 g/l. Zu den festen Darreichungsformen des Mittels zählen ferner Extrudate, Granulate, Tabletten oder Pouches enthaltend feste Mittel, die sowohl in Großgebinden als auch portionsweise abgepackt vorliegen können. Alternativ kann das Mittel auch flüssig, gelförmig oder pastös sein, z.B. in Form eines nicht-wässrigen Mittels oder einer nicht-wässrigen Paste oder in Form eines wässrigen Mittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen (Mehrkomponentensystem). Ein solches Mittel ist demnach in mehrere Komponenten aufgeteilt, z.B. zwei flüssige, zwei feste oder eine flüssige und eine feste Phase. Die flüssigen Angebotsformen auf Basis von Wasser und/oder organischen Lösungsmitteln können verdickt, in Form von Gelen vorliegen.

Ein Stoff, z.B. eine Zusammensetzung oder ein Mittel ist gemäß Definition der Erfindung festförmig, wenn sie bei 25°C und 1.013 mbar im festen Aggregatzustand vorliegt.

Ein Stoff, z.B. eine Zusammensetzung oder ein Mittel ist gemäß Definition der Erfindung flüssig, wenn sie bei 25°C und 1.013 mbar im flüssigen Aggregatzustand vorliegt. Dabei umfasst flüssig auch gelförmig.

Wenn die erfindungsgemäßen Waschmittel in flüssiger Form vorliegen, enthalten sie vorzugsweise bezogen auf ihr Gesamtgewicht mehr als 40 Gew.-%, vorzugsweise 50 bis 90 Gew.-% und besonders bevorzugt 60 bis 80 Gew.-% Wasser.

Einen weiteren Erfindungsgegenstand stellt ein Verfahren zur Reinigung von Textilien dar, wobei in mindestens einem Verfahrensschritt ein erfindungsgemäßes Textilwaschmittel, insbesondere ein flüssiges Textilwaschmittel, angewendet wird. Hierunter fallen sowohl manuelle als auch maschinelle Verfahren, wobei maschinelle Verfahren aufgrund ihrer präziseren Steuerbarkeit, was z.B. die eingesetzten Mengen und Einwirkzeiten angeht, bevorzugt sind. Verfahren zur Reinigung von Textilien zeichnen sich im Allgemeinen dadurch aus, dass in einem oder mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung dieses Mittels behandelt wird.

In verschiedenen Ausführungsformen zeichnet sich das Verfahren dadurch aus, dass die Protease bei einer Temperatur von 0 bis 100°C, bevorzugt 10 bis 70°C, weiter bevorzugt 30 bis 50°C und am meisten bevorzugt bei 20 bis 40°C eingesetzt wird. Insbesondere werden in Verfahren, welche bei einer Temperatur zwischen 10 und 60°C, bevorzugt zwischen 15 und 50°C und weiter bevorzugt zwischen 20 und 40°C durchgeführt werden, die Vorteile beim Einsatz der erfindungsgemäßen Textilwaschmittel gegenüber Textilwaschmittel mit herkömmlich eingesetzten Proteasen für den Verbraucher sichtbar.

Alle Sachverhalte, Gegenstände und Ausführungsformen, die für erfindungsgemäße textile Waschmittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Die in erfindungsgemäßen Mitteln eingesetzten Proteasen sind entsprechend der vorstehenden Ausführungen in Textilwaschmitteln sowie Verfahren zur Textilwäsche vorteilhaft einsetzbar. Sie können also vorteilhaft dazu verwendet werden, um in entsprechenden Mitteln und Verfahren eine proteolytische Aktivität bereitzustellen.

Schließlich erfasst die Erfindung auch die Verwendung der hierin beschriebenen Proteasen in Waschmitteln, beispielsweise wie oben beschrieben, zur (verbesserten) Entfernung von proteasesensitiven Anschmutzungen, beispielsweise von Textilien. In bevorzugten Ausführungsformen dieser Verwendung wird die Protease im Waschmittel vor einem Waschvorgang 3 oder mehr Tage, 4 oder mehr Tage, 7 oder mehr Tage, 10 oder mehr Tage, 12 oder mehr Tage, 14 oder mehr Tage, 21 oder mehr Tage oder 28 oder mehr Tage gelagert.

Weitere Erfindungsgegenstände sind daher
- eine Verwendung eines erfindungsgemäßen Mittels zur Reinigung von Textilien, und/oder
- eine Verwendung einer Protease, die eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an den Positionen, die den Positionen 9, 130, 133, 144, 217, 252 und 271 entsprechen, Aminosäuresubstitutionen, insbesondere die Aminosäuresubstitutionen 9T, 130D/V, 133A, 144K, 217M, 252T und 271E, und (ii) an mindestens einer, vorzugsweise mindestens zwei, der Positionen, die den Positionen 6, 89, 131, 166, 189, 211 oder 224 entsprechen, mindestens eine weitere Aminosäuresubstitution, insbesondere ausgewählt aus 6W/F, 89A/G, 131H/Y/F, 166M/L/I, 189T/L/I, 211N/Q und 224A/G, bevorzugter ausgewählt aus 6W, 89A, 131H, 166M, 189T, 211N und 224A, aufweist, in einem Textilwaschmittel, vorzugsweise einem flüssigen Textilwaschmittel, zur Entfernung von proteasesensitiven Anschmutzungen auf Textilien, wobei das Textilwaschmittel einen pH-Wert von etwa 9 bis etwa 12, gemessen in 1 Gew.-%iger Lösung in entionisiertem Wasser bei 20°C aufweist,
insbesondere derart, dass die Protease jeweils in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro Gramm des Mittels eingesetzt wird.

Besonders bevorzugte Ausführungsformen stellen dabei z.B. die Handwäsche, die manuelle Entfernung von Flecken von Textilien oder die Verwendung im Zusammenhang mit einem maschinellen Verfahren dar.

Alle Sachverhalte, Gegenstände und Ausführungsformen, die für erfindungsgemäße Proteasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

### Bestimmung der Lagerstabilität in flüssigem Waschmittel

### Verwendete Waschmittelmatrix

Es wurde eine handelsübliche flüssige Waschmittel, wie in Tabelle 1 angegeben, verwendet.

**Tabelle 1: Formula A**

| Inhaltsstoff | Formula A (in Gew.-%) |
|---|---|
| LAS (anionisches Tensid) | 3% |
| Alcohol Ethoxylate 7EO | 2% |
| Alcohol Ether Sulfate 3EO | 8% |
| Kokosnussfettsäuren | 0,5% |
| NaOH 50% | 0,4% |
| Natriumcarbonat | 3% |
| Distyrlbiphenyldisulfonat | 0,05% |
| Duftstoffe | 0,5% |
| Wasser demin. & Misc. | |

Der pH-Wert von Formula A wurde mit Citronensäure auf pH 10 eingestellt, gemessen in 1 Gew.- %-iger Lösung in entionisiertem Wasser bei 20°C.

Als Proteasen wurden verwendet:
V1: Protease gemäß SEQ ID NO:2 aus WO 2013/060621 A1
V2: Subtilisin 309 aus *Bacillus lentus*
E1: SEQ ID NO:1+P9T+S89A+N130D+T133A+N144K+S189T+Y217M+S224A+N252T+Q271E

### Lagerung

Die Proteasen liegen in Bioreaktor generierten Überständen in *Bacillus licheniformis* vor. Sie werden auf ein gleiches Aktivitätslevel verdünnt. Es wurden 90% Waschmittelmatrix mit 10% entsprechend verdünnten *Bacillus licheniformis* Überstand versetzt und gut gemischt. Die Enzymaktivität in jedem Ansatz wurde bestimmt (Startwert), bevor die verschlossenen Gefäße wurden für zwei Wochen bei 30°C bzw. 40°C gelagert. Die entnommene Probenmenge wurde für 20 min bei Raumtemperatur in 0,1M Tris/HCl (pH 8,6) durch Rühren gelöst. Danach wurde der AAPF Assay wie unten beschrieben durchgeführt.

### Protease-Aktivitäts-Assay (AAPF Assay)

Die Aktivität der Protease wird durch die Freisetzung des Chromophors para-Nitroanilin aus dem Substrat Succinyl Alanin-Alanin-Prolin-Phenylalanin-para-Nitroanilid (AAPFpNA; Bachem L-1400) bestimmt. Die Freisetzung des pNA verursacht eine Zunahme der Extinktion bei 410 nm, deren zeitlicher Verlauf ein Maß für die enzymatische Aktivität ist.

Die Messung erfolgte bei einer Temperatur von 25°C, pH 8,6 und einer Wellenlänge von 410 nm. Die Messzeit betrug 5 min bei einem Messintervall von 20 bis 60 Sekunden.

### Messansatz:

10 µL AAPF-Lösung (70 mg/mL)
1000 µL Tris/HCl (0,1 M; pH 8,6 mit 0,1 % Brij 35)
10 µL verdünnte Protease-Lösung
Kinetik erstellt über 5 min bei 25°C (410 nm)

### Ergebnisse

Im Folgenden ist die Restaktivität in % nach Lagerung in o.g. Waschmittelmatrix gezeigt. Die Restaktivität in % ist bezogen auf die jeweilige Enzymaktivität vor Lagerung (Startwert).

**Tabelle 2: Lagerung in Formula B (pH 10) bei 30°C bzw. 40°C für zwei Wochen**

| Protease | Restaktivität (%) | |
|---|---|---|
| | 30°C | 40°C |
| V1 | 18 | 3 |
| V2 | 9 | 0 |
| E1 | 33 | 6 |

Die erfindungsgemäße Protease E1 zeigt in einer hochalkalischen Waschmittelformulierung nach Lagerung für 2 Wochen eine deutliche höhere Lagerstabilität als die Vergleichsproteasen V1 und V2.

## Patentansprüche

1. Textilwaschmittel, insbesondere flüssiges Textilwaschmittel, umfassend
a) mindestens eine Protease, wobei die Protease proteolytische Aktivität aufweist und eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an den Positionen, die den Positionen 9, 130, 133, 144, 217, 252 und 271 entsprechen, Aminosäuresubstitutionen, insbesondere die Aminosäuresubstitutionen 9T, 130D/V, 133A, 144K, 217M, 252T und 271E, und (ii) an mindestens einer, vorzugsweise mindestens zwei, der Positionen, die den Positionen 6, 89, 131, 166, 189, 211 oder 224 entsprechen, mindestens eine weitere Aminosäuresubstitution, insbesondere ausgewählt aus 6W/F, 89A/G, 131H/Y/F, 166M/L/I, 189T/L/I, 211N/Q und 224A/G, bevorzugter ausgewählt aus 6W, 89A, 131H, 166M, 189T, 211N und 224A, aufweist, und
b) mindestens einen Waschmittelinhaltsstoff, vorzugsweise in einer Menge von 0,01 bis 99,9 Gew.-%,
wobei das Textilwaschmittel einen pH-Wert von etwa 9 bis etwa 12, gemessen in 1 Gew.- %iger Lösung in entionisiertem Wasser bei 20°C aufweist.

2. Waschmittel nach Anspruch 1, wobei die Protease eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96% und 96,5% identisch ist, und jeweils bezogen auf die Nummerierung gemäß SEQ ID NO:1 (i) an den Positionen, die den Positionen 9, 130, 133, 144, 217, 252 und 271 entsprechen, die Aminosäuresubstitutionen 9T, 130D/V, 133A, 144K, 217M, 252T und 271E, und (ii) an mindestens zwei der Positionen, die den Positionen 6, 89, 131, 166, 189, 211 oder 224 entsprechen, mindestens zwei weitere Aminosäuresubstitutionen, die aus der aus 6W/F, 89A/G, 131H/Y/F, 166M/L/I, 189T/L/I, 211N/Q und 224A/G, vorzugsweise 6W, 89A, 131H, 166M, 189T, 211N und 224A, bestehenden Gruppe ausgewählt sind, aufweist.

3. Waschmittel nach Anspruch 1 oder 2, wobei die Protease eine der folgenden Aminosäuresubstitutionsvarianten, jeweils bezogen auf die Nummerierung gemäß SEQ ID NO:1, aufweist:
(i) P9T+N130D+T133A+N144K+G166M+S189T+Y217M+N252T+Q271E;
(ii) P9T+N130D+T133A+N144K+G166M+S189T+Y217M+S224A+N252T+Q271E;
(iii) P9T+S89A+N130D+G131H+T133A+N144K+S189T+Y217M+S224A+N252T+Q271E;
(iv) Y6W+P9T+N130D+T133A+N144K+S189T+Y217M+S224A+N252T+Q271E;
(v) P9T+N130D+T133A+N144K+G166M+S211N+Y217M+N252T+Q271E;
(vi) P9T+S89A+N130D+T133A+N144K+S189T+Y217M+S224A+N252T+Q271E.

4. Waschmittel nach einem der Ansprüche 1 bis 3, wobei die Protease in einer Menge von 1 x 10⁻⁸ bis 5 Gew.-% und zunehmend bevorzugt von 0,0001 bis 1 Gew.-%, von 0,0005 bis 0,5 Gew.-%, von 0,001 bis 0,1 Gew.-%, bezogen auf das Gesamtgewicht des Waschmittels in diesem enthalten ist.

5. Waschmittel nach einem der Ansprüche 1 bis 4, wobei das Mittel mindestens ein weiteres Enzym umfasst, das aus der aus Amylasen, Cellulasen, Hemicellulasen, Mannanasen, Tannasen, Xylanasen, Xanthanasen, Xyloglucanasen, β-Glucosidasen, Pektinasen, Carrageenasen, Perhydrolasen, Oxidasen, Oxidoreduktasen, Lipase und Kombinationen davon bestehenden Gruppe ausgewählt ist, vorzugsweise mindestens eine Amylase.

6. Waschmittel nach Anspruch 5, wobei das mindestens eine weitere Enzym eine Amylase ist und wobei die Amylase ausgewählt ist aus:
a) einer α-Amylase, die eine Aminosäuresequenz umfasst, die zu der in SEQ ID NO:2 angegebenen Aminosäuresequenz über deren Gesamtlänge zu mindestens 80% identisch ist und optional mindestens eine Aminosäuresubstitution an einer der Positionen 172, 202, 208, 255 und 261 in der Zählung gemäß SEQ ID NO:2 aufweist, vorzugsweise ausgewählt aus der aus M202L, M202V, M202S, M202T, M202I, M202Q, M202W, S255N, R172Q und Kombinationen davon bestehenden Gruppe; und/oder
b) einer α-Amylase, die eine Aminosäuresequenz umfasst, die zu der in SEQ ID NO:3 angegebenen Aminosäuresequenz über deren Gesamtlänge zu mindestens 60% identisch ist und optional mindestens eine Aminosäuresubstitution an einer der Positionen 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 und 484 und/oder eine Deletion an einer der Positionen 183 und 184 in der Zählung gemäß SEQ ID NO:3 aufweist, vorzugsweise mindestens eine Aminosäuresubstitution an einer der Positionen 9, 26, 149, 182, 186, 202, 257, 295, 299, 323, 339 and 345, und/oder besonders bevorzugt mindestens eine Aminosäuresubstitution oder -deletion, die aus der aus R118K, D183*, G184*, N195F, R320K, R458K und Kombinationen davon bestehenden Gruppe ausgewählt ist; und/oder
c) einer α-Amylase, die eine Aminosäuresequenz umfasst, die zu der in SEQ ID NO:4 angegebenen Aminosäuresequenz über deren Gesamtlänge zu mindestens 90% identisch ist und optional mindestens eine Substitution und/oder Deletion an einer der Positionen 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 und 477 in der Zählung gemäß SEQ ID NO:4, aufweist, vorzugsweise Aminosäuredeletionen an den Positionen 183 und 184.

7. Waschmittel nach einem der Ansprüche 1 bis 6, wobei der Waschmittelinhaltsstoff aus der aus Tensiden, Buildern, Komplexbildnern, Polymeren, Glaskorrosionsinhibitoren, Korrosionsinhibitoren, Bleichmitteln wie Persauerstoffverbindungen, Bleichaktivatoren oder Bleichkatalysatoren, wassermischbaren organischen Lösungsmitteln, Enzymstabilisatoren, Sequestrierungsmitteln, Elektrolyten, pH-Regulatoren und/oder weiteren Hilfsstoffen wie optischen Aufhellern, Vergrauungsinhibitoren, Farbübertragungsinhibitoren, Schaumregulatoren sowie Farb- und Duftstoffen sowie Kombinationen davon bestehenden Gruppe ausgewählt ist.

8. Waschmittel nach einem der Ansprüche 1 bis 7, umfassend
a) 5 bis 20 Gew.-%, insbesondere 9 bis 17 Gew.-% anionische Tenside und/oder
b) 1 bis 20 Gew.-%, insbesondere 5 bis 9 Gew.-% nichtionische Tenside und/oder
c) 0,5 bis 10 Gew.-%, insbesondere 1 bis 4 Gew.-%, wasserlösliche organische Buildersubstanzen, insbesondere Citronensäure oder Citrat, und/oder
d) 0,01 bis 5 Gew.-%, insbesondere 0,5 bis 2 Gew.-% Komplexbildner, insbesondere HEDP, und/oder
e) 0,01 bis 5 Gew.-%, insbesondere 0,2 bis 1 Gew.-% Vergrauungsinhibitor und/oder
f) 0,01 bis 5 Gew.-% Schauminhibitor.

9. Waschmittel nach einem der Ansprüche 1 bis 7, umfassend
a) 0 bis 10 Gew.-%, insbesondere 1 bis 3 Gew.-% anionische Tenside und/oder
b) 5 bis 20 Gew.-%, insbesondere 10 bis 18 Gew.-% nichtionische Tenside und/oder
c) 1 bis 10 Gew.-%, insbesondere 1,5 bis 3 Gew.-% wasserlösliches oder wasserdispergierbares anorganisches Buildermaterial und/oder
d) 0 bis 1 Gew.-%, insbesondere 0,01 bis 0,5 Gew.-% Duftstoffe und/oder
e) 0,01 bis 5 Gew.-%, insbesondere 0,05 bis 0,5 Gew.-% optische Aufheller.

10. Waschmittel nach einem der Ansprüche 1 bis 9, wobei es in 1 Gew.-%iger Lösung in entionisiertem Wasser bei 20°C einen pH-Wert in einem Bereich von etwa 9 bis etwa 12, insbesondere von etwa 9,5 bis etwa 11,5, weiter bevorzugt von etwa 10 bis etwa 11, besonders bevorzugt von etwa pH 10 aufweist.

11. Verfahren zur Reinigung von Textilien, **dadurch gekennzeichnet, dass** in mindestens einem Verfahrensschritt ein Mittel nach einem der Ansprüche 1 bis 10 angewendet wird.

12. Verwendung eines Waschmittels nach einem der Ansprüche 1 bis 10 zur Entfernung von proteasesensitiven Anschmutzungen von Textilien.

13. Verwendung einer Protease, die eine Aminosäuresequenz umfasst, die mindestens 70% Sequenzidentität mit der in SEQ ID NO:1 angegebenen Aminosäuresequenz über deren Gesamtlänge aufweist und jeweils bezogen auf die Nummerierung gemäß SEQ ID NO:1 (i) an den Positionen, die den Positionen 9, 130, 133, 144, 217, 252 und 271 entsprechen, Aminosäuresubstitutionen, insbesondere die Aminosäuresubstitutionen 9T, 130D/V, 133A, 144K, 217M, 252T und 271E, und (ii) an mindestens einer, vorzugsweise mindestens zwei, der Positionen, die den Positionen 6, 89, 131, 166, 189, 211 oder 224 entsprechen, mindestens eine weitere Aminosäuresubstitution, insbesondere ausgewählt aus 6W/F, 89A/G, 131H/Y/F, 166M/L/I, 189T/L/I, 211N/Q und 224A/G, bevorzugter ausgewählt aus 6W, 89A, 131H, 166M, 189T, 211N und 224A, aufweist, in einem Textilwaschmittel, vorzugsweise einem flüssigen Textilwaschmittel, zur Entfernung von proteasesensitiven Anschmutzungen auf Textilien, wobei das Textilwaschmittel einen pH-Wert von etwa 9 bis etwa 12, gemessen in 1 Gew.-%-iger Lösung in entionisiertem Wasser bei 20°C aufweist.

## Claims

1. Textile detergent, in particular liquid textile detergent, comprising
a) at least one protease, wherein the protease exhibits proteolytic activity and comprises an amino acid sequence that exhibits at least 70% sequence identity with the amino acid sequence specified in SEQ ID NO:1 over its entire length and, in each case, relative to the numbering according to SEQ ID NO:1 (i) at the positions corresponding to positions 9, 130, 133, 144, 217, 252, and 271, amino acid substitutions, in particular the amino acid substitutions 9T, 130D/V, 133A, 144K, 217M, 252T and 271E, and (ii) at at least one, preferably at least two, of the positions corresponding to positions 6, 89, 131, 166, 189, 211 or 224, at least one further amino acid substitution, in particular selected from 6W/F, 89A/G, 131H/Y/F, 166M/L/I, 189T/L/I, 211N/Q and 224A/G, preferably selected from 6W, 89A, 131H, 166M, 189T, 211N, and 224A, and
b) at least one detergent ingredient, preferably in an amount of 0.01 to 99.9 % by weight,
wherein the textile detergent has a pH value about 9 to about 12, measured in a 1 % by weight solution in deionized water at 20°C.

2. Detergent according to claim 1, wherein the protease comprises an amino acid sequence that is at least 70% and increasingly preferably at least 71%, 72%, 73%, 74%, 75%, 76%, 77%, 78%, 79%, 80%, 81%, 82%, 83%, 84%, 85%, 86%, 87%, 88%, 89%, 90%, 90.5%, 91%, 91.5%, 92%, 92.5%, 93%, 93.5%, 94%, 94.5%, 95%, 95.5%, 96%, and 96.5%, and in each case, based on the numbering according to SEQ ID NO:1 (i), at the positions corresponding to positions 9, 130, 133, 144, 217, 252 and 271, the amino acid substitutions 9T, 130D/V, 133A, 144K, 217M, 252T and 271E, and (ii) at at least two of the positions corresponding to positions 6, 89, 131, 166, 189, 211, or 224, at least two further amino acid substitutions selected from the group consisting of 6W/F, 89A/G, 131H/Y/F, 166M/L/I, 189T/L/I, 211N/Q, and 224A/G, preferably 6W, 89A, 131H, 166M, 189T, 211N and 224A, preferably 6W, 89A, 131H, 166M, 189T, 211N and 224A.

3. Detergent according to claim 1 or 2, wherein the protease has one of the following amino acid substitution variants, each based on the numbering according to SEQ ID NO:1:
(i) P9T+N130D+T133A+N144K+G166M+S189T+Y217M+N252T+Q271E;
(ii) P9T+N130D+T133A+N144K+G166M+S189T+Y217M+S224A+N252T+Q271E;
(iii) P9T+S89A+N130D+G131H+T133A+N144K+S189T+Y217M+S224A+N252T+Q271E;
(iv) Y6W+P9T+N130D+T133A+N144K+S189T+Y217M+S224A+N252T+Q271E;
(v) P9T+N130D+T133A+N144K+G166M+S211N+Y217M+N252T+Q271E;
(vi) P9T+S89A+N130D+T133A+N144K+S189T+Y217M+S224A+N252T+Q271E.

4. **Detergent** according to any one of claims 1 to 3, wherein the protease is present in an amount of 1 x 10⁻⁸ to 5 % by weight and increasingly preferably from 0.0001 to 1 % by weight, from 0.0005 to 0.5 % by weight, from 0.001 to 0.1 % by weight, based on the total weight of the detergent contained therein.

5. Detergent according to any one of claims 1 to 4, wherein the agent comprises at least one further enzyme selected from the group consisting of amylases, cellulases, hemicellulases, mannanases, tannases, xylanases, xanthanases, xyloglucanases, β-glucosidases, pectinases, carrageenases, perhydrolases, oxidases, oxidoreductases, lipase, and combinations thereof, preferably at least one amylase.

6. Detergent according to claim 5, wherein the at least one further enzyme is an amylase and wherein the amylase is selected from:
a) α -amylase comprising an amino acid sequence that is at least 80% identical to the amino acid sequence specified in SEQ ID NO:2 over its entire length and optionally has at least one amino acid substitution at one of positions 172, 202, 208, 255, and 261 in the numbering according to SEQ ID NO:2, preferably selected from the group consisting of M202L, M202V, M202S, M202T, M202I, M202Q, M202W, S255N, R172Q, and combinations thereof; and/or
b) α-amylase comprising an amino acid sequence that is at least 60% identical to the amino acid sequence specified in SEQ ID NO:3 over its entire length and optionally has at least one amino acid substitution at one of positions 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 and 484 and/or a deletion at one of positions 183 and 184 in the counting according to SEQ ID NO:3, preferably at least one amino acid substitution at one of positions 9, 26, 149, 182, 186, 202, 257, 295, 299, 323, 339, and 345, and/or particularly preferably at least one amino acid substitution or deletion selected from the group consisting of R118K, D183*, G184*, N195F, R320K, R458K, and combinations thereof; and/or
c) α-amylase comprising an amino acid sequence that is at least 90% identical to the amino acid sequence specified in SEQ ID NO:4 over its entire length and optionally has at least one substitution and/or deletion at one of positions 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476, and 477 in the numbering according to SEQ ID NO:4, preferably amino acid deletions at positions 183 and 184.

7. Detergent according to any one of claims 1 to 6, wherein the detergent ingredient is selected from the group consisting of surfactants, builders, complexing agents, polymers, glass corrosion inhibitors, corrosion inhibitors, bleaching agents such as peroxygen compounds, bleach activators or bleach catalysts, water-miscible organic solvents, enzyme stabilizers, sequestrants, electrolytes, pH regulators, and/or other auxiliaries such as optical brighteners, gray inhibitors, color transfer inhibitors, foam regulators, and dyes and fragrances, as well as combinations thereof.

8. Detergent according to any of claims 1 to 7, comprising
a) 5 to 20 % by weight, in particular 9 to 17 % by weight, of anionic surfactants and/or
b) 1 to 20 % by weight, in particular 5 to 9 % by weight, of nonionic surfactants and/or
c) 0.5 to 10 % by weight, in particular 1 to 4 % by weight, water-soluble organic builder substances, in particular citric acid or citrate, and/or
d) 0.01 to 5 % by weight, in particular 0.5 to 2 % by weight, of complexing agents, in particular HEDP, and/or
e) 0.01 to 5 % by weight, in particular 0.2 to 1 % by weight, of a gray inhibitor and/or
f) 0.01 to 5 % by weight of a foam inhibitor.

9. Detergent according to any one of claims 1 to 7, comprising
a) 0 to 10 % by weight, in particular 1 to 3 % by weight, of anionic surfactants and/or
b) 5 to 20 % by weight, in particular 10 to 18 % by weight, of nonionic surfactants and/or
c) 1 to 10 % by weight, in particular 1.5 to 3 % by weight, of water-soluble or water-dispersible inorganic builder material and/or
d) 0 to 1 % by weight, in particular 0.01 to 0.5 % by weight, of fragrances and/or
e) 0.01 to 5 % by weight, in particular 0.05 to 0.5 % by weight, optical brighteners.

10. Detergent according to one of claims 1 to 9, wherein it has a pH value in a range of about 9 to about 12, in particular from about 9.5 to about 11.5, more preferably from about 10 to about 11, particularly preferably from about pH 10, in a 1 % by weight solution in deionized water at 20°C.

11. Method for cleaning textiles, **characterized in that** an agent according to one of claims 1 to 10 is used in at least one step of the method.

12. Use of a detergent according to one of claims 1 to 10 for removing protease-sensitive soiling from textiles.

13. Use of a protease comprising an amino acid sequence which has at least 70% sequence identity with the amino acid sequence specified in SEQ ID NO:1 over its entire length and, in each case, based on the numbering according to SEQ ID NO:1 (i) at the positions corresponding to positions 9, 130, 133, 144, 217, 252, and 271, amino acid substitutions, in particular the amino acid substitutions 9T, 130D/V, 133A, 144K, 217M, 252T and 271E, and (ii) at at least one, preferably at least two, of the positions corresponding to positions 6, 89, 131, 166, 189, 211 or 224, at least one further amino acid substitution, in particular selected from 6W/F, 89A/G, 131H/Y/F, 166M/L/I, 189T/L/I, 211N/Q and 224A/G, preferably selected from 6W, 89A, 131H, 166M, 189T, 211N and 224A, in a textile detergent, preferably a liquid textile detergent, for removing protease-sensitive soiling from textiles, wherein the textile detergent has a pH value of about 9 to about 12, measured in a 1 % by weight solution in deionized water at 20°C.

## Revendications

1. Détergent textile, en particulier détergent textile liquide, comprenant
a) au moins une protéase, la protéase présentant une activité protéolytique et comprenant une séquence d'acides aminés qui présente une identité de séquence d'au moins 70 % avec la séquence d'acides aminés indiquée dans SEQ ID NO:1 sur toute sa longueur et, par rapport à la numérotation selon SEQ ID NO:1, respectivement (i) aux positions qui correspondent aux positions 9, 130, 133, 144, 217, 252 et 271, des substitutions d'acides aminés, en particulier les substitutions d'acides aminés 9T, 130D/V, 133A, 144K, 217M, 252T et 271E, et (ii) à au moins une, de préférence au moins deux, des positions correspondant aux positions 6, 89, 131, 166, 189, 211 ou 224, au moins une autre substitution d'acide aminé, en particulier choisie parmi 6W/F, 89A/G, 131H/Y/F, 166M/L/I, 189T/L/I, 211N/Q et 224A/G, de préférence choisie parmi 6W, 89A, 131H, 166M, 189T, 211N et 224A, et
b) au moins un ingrédient détergent, de préférence en une quantité de 0,01 à 99,9 % en poids,
le détergent textile ayant un pH d'environ 9 à environ 12, mesuré dans une solution à 1 % en poids dans de l'eau désionisée à 20 °C.

2. Détergent selon la revendication 1, dans lequel la protéase comprend une séquence d'acides aminés qui correspond à la séquence d'acides aminés indiquée dans SEQ ID NO:1 sur au moins 70 % de sa longueur totale et, de manière préférentielle, sur au moins 71 %, 72 %, 73 %, 74 %, 75 %, 76 %, 77 %, 78 %, 79 %, 80 %, 81 %, 82 %, 83 %, 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 % et 96,5 %, et, par rapport à la numérotation selon SEQ ID NO:1 (i), aux positions correspondant aux positions 9, 130, 133, 144, 217, 252 et 271, les substitutions d'acides aminés 9T, 130D/V, 133A, 144K, 217M, 252T et 271E, et (ii) à au moins deux des positions correspondant aux positions 6, 89, 131, 166, 189, 211 ou 224, au moins deux autres substitutions d'acides aminés choisies parmi le groupe constitué de 6W/F, 89A/G, 131H/Y/F, 166M/L/I, 189T/L/I, 211N/Q et 224A/G, de préférence 6W, 89A, 131H, 166M, 189T, 211N et 224A.

3. Détergent selon la revendication 1 ou 2, dans lequel la protéase présente l'une des variantes de substitution d'acides aminés suivantes, chacune se rapportant à la numérotation selon SEQ ID NO:1 :
(i) P9T+N130D+T133A+N144K+G166M+S189T+Y217M+N252T+Q271E ;
(ii) P9T+N130D+T133A+N144K+G166M+S189T+Y217M+S224A+N252T+Q271E ;
(iii) P9T+S89A+N130D+G131H+T133A+N144K+S189T+Y217M+S224A+N252T+Q271E ;
(iv) Y6W+P9T+N130D+T133A+N144K+S189T+Y217M+S224A+N252T+Q271E ;
(v) P9T+N130D+T133A+N144K+G166M+S211N+Y217M+N252T+Q271E ;
(vi) P9T+S89A+N130D+T133A+N144K+S189T+Y217M+S224A+N252T+Q271E.

4. Détergent selon l'une des revendications 1 à 3, dans lequel la protéase est présente en une quantité de 1 x 10⁻⁸ à 5 % en poids et, de manière préférentielle, de 0,0001 à 1 % en poids, de 0,0005 à 0,5 % en poids, de 0,001 à 0,1 % en poids, par rapport au poids total du détergent dans lequel il est contenu.

5. Détergent selon l'une des revendications 1 à 4, dans lequel le produit comprend au moins une autre enzyme choisie parmi les amylases, les cellulases, les hémicellulases, les mannanases, les tannases, les xylanases, les xanthannases, xyloglucanases, ß-glucosidases, pectines, carragénases, perhydrolases, oxydases, oxydoréductases, lipases et leurs combinaisons, de préférence au moins une amylase.

6. Détergent selon la revendication 5, dans lequel ladite au moins une autre enzyme est une amylase et dans lequel ladite amylase est choisie parmi :
a) Une α-amylase, qui comprend une séquence d'acides aminés qui est identique à au moins 80 % à la séquence d'acides aminés indiquée dans SEQ ID NO:2 sur toute sa longueur et qui présente éventuellement au moins une substitution d'acide aminé à l'une des positions 172, 202, 208, 255 et 261 dans le comptage selon SEQ ID NO:2, de préférence choisie dans le groupe constitué de M202L, M202V, M202S, M202T, M202I, M202Q, M202W, S255N, R172Q et leurs combinaisons ; et/ou
b) une α-amylase qui comprend une séquence d'acides aminés qui est identique à au moins 60 % à la séquence d'acides aminés indiquée dans SEQ ID NO:3 sur toute sa longueur et qui comporte éventuellement au moins une substitution d'acide aminé à l'une des positions 9, 26, 30, 33, 82, 37, 106, 118, 128, 133, 149, 150, 160, 178, 182, 186, 193, 195, 202, 203, 214, 231, 256, 257, 258, 269, 270, 272, 283, 295, 296, 298, 299, 303, 304, 305, 311, 314, 315, 318, 319, 320, 323, 339, 345, 361, 378, 383, 419, 421, 437, 441, 444, 445, 446, 447, 450, 458, 461, 471, 482 et 484 et/ou une délétion à l'une des positions 183 et 184 dans le décompte selon SEQ ID NO:3, de préférence au moins une substitution d'acide aminé à l'une des positions 9, 26, 149, 182, 186, 202, 257, 295, 299, 323, 339 et 345, et/ou de préférence au moins une substitution ou délétion d'acide aminé choisie dans le groupe constitué par R118K, D183*, G184*, N195F, R320K, R458K et leurs combinaisons ; et/ou
c) α-amylase qui comprend une séquence d'acides aminés qui est identique à au moins 90 % à la séquence d'acides aminés indiquée dans SEQ ID NO:4 sur toute sa longueur et qui comporte éventuellement au moins une substitution et/ou une délétion à l'une des positions 93, 116, 118, 129, 133, 134, 140, 142, 146, 147, 149, 151, 152, 169, 174, 183, 184, 186, 189, 193, 195, 197, 198, 200, 203, 206, 210, 212, 213, 235, 243, 244, 260, 262, 284, 303, 304, 320, 338, 347, 359, 418, 431, 434, 439, 447, 458, 469, 476 et 477 dans la numérotation selon SEQ ID NO:4, de préférence des délétions d'acides aminés aux positions 183 et 184.

7. Détergent selon l'une des revendications 1 à 6, dans lequel l'ingrédient détergent est choisi parmi les tensioactifs, les adjuvants, les agents complexants, les polymères, les inhibiteurs de corrosion du verre, les inhibiteurs de corrosion, les agents de blanchiment tels que les composés peroxygénés, les activateurs de blanchiment ou les catalyseurs de blanchiment, les solvants organiques miscibles à l'eau, les stabilisateurs d'enzymes, les agents séquestrants, électrolytes, régulateurs de pH et/ou d'autres adjuvants tels que des azurants optiques, des inhibiteurs de grisaillement, des inhibiteurs de transfert de couleur, des régulateurs de mousse ainsi que des colorants et des parfums, et de leurs combinaisons.

8. Détergent selon l'une des revendications 1 à 7, comprenant
a) 5 à 20 % en poids, en particulier 9 à 17 % en poids, de tensioactifs anioniques et/ou
b) 1 à 20 % en poids, en particulier 5 à 9 % en poids, de tensioactifs non ioniques et/ou
c) 0,5 à 10 % en poids, en particulier 1 à 4 % en poids, de substances adjuvantes organiques hydrosolubles, en particulier d'acide citrique ou de citrate, et/ou
d) 0,01 à 5 % en poids, en particulier 0,5 à 2 % en poids d'agents complexants, en particulier HEDP, et/ou
e) 0,01 à 5 % en poids, en particulier 0,2 à 1 % en poids, d'inhibiteur de grisaillement et/ou
f) 0,01 à 5 % en poids d'inhibiteur de mousse.

9. Détergent selon l'une des revendications 1 à 7, comprenant
a) 0 à 10 % en poids, en particulier 1 à 3 % en poids, de tensioactifs anioniques et/ou
b) 5 à 20 % en poids, en particulier 10 à 18 % en poids, de tensioactifs non ioniques et/ou
c) 1 à 10 % en poids, en particulier 1,5 à 3 % en poids, de matière adjuvante inorganique soluble dans l'eau ou dispersable dans l'eau et/ou
d) 0 à 1 % en poids, en particulier 0,01 à 0,5 % en poids de parfums et/ou
e) 0,01 à 5 % en poids, en particulier 0,05 à 0,5 % en poids d'azurants optiques.

10. Détergent selon l'une des revendications 1 à 9, qui, dans une solution à 1 % en poids dans de l'eau désionisée à 20 °C, présente un pH compris entre environ 9 et environ 12, en particulier entre environ 9,5 et environ 11,5, de préférence entre environ 10 et environ 11, et de manière particulièrement préférée autour de pH 10.

11. Procédé de nettoyage de textiles, **caractérisé en ce qu'**un agent selon l'une des revendications 1 à 10 est utilisé dans au moins une étape du procédé.

12. Utilisation d'un détergent selon l'une des revendications 1 à 10 pour éliminer les salissures sensibles aux protéases des textiles.

13. Utilisation d'une protéase qui comprend une séquence d'acides aminés présentant au moins 70 % d'identité de séquence avec la séquence d'acides aminés indiquée dans SEQ ID NO:1 sur toute sa longueur et, par rapport à la numérotation selon SEQ ID NO:1 (i) aux positions correspondant aux positions 9, 130, 133, 144, 217, 252 et 271, des substitutions d'acides aminés, en particulier les substitutions d'acides aminés 9T, 130D/V, 133A, 144K, 217M, 252T et 271E, et (ii) à au moins une, de préférence au moins deux, des positions correspondant aux positions 6, 89, 131, 166, 189, 211 ou 224, au moins une autre substitution d'acide aminé, en particulier choisie parmi 6W/F, 89A/G, 131H/Y/F, 166M/L/I, 189T/L/I, 211N/Q et 224A/G, de préférence choisie parmi 6W, 89A, 131H, 166M, 189T, 211N et 224A, dans un détergent textile, de préférence un détergent textile liquide, pour éliminer les salissures sensibles aux protéases sur les textiles, le détergent textile ayant un pH d'environ 9 à environ 12, mesuré dans une solution à 1 % en poids dans de l'eau désionisée à 20 °C.
